# EUROPEAN PATENT APPLICATION

(11) **EP 4 406 939 A1**
(43) Date of publication of application: **31.07.2024**
(21) Application number: 23305100.2
(22) Date of filing: 26.01.2023
(51) Int. Cl.: C07D 233/64, C07D 235/12, C07D 263/32, C07D 277/24, A61P 35/00, A61K 31/4164

(54) **CALIX[4]ARENES WITH HIGH ANTICANCER ACTIVITY**

(71) Applicant: Université Grenoble Alpes, 38400 Saint-Martin-d'Hères (FR); Université Libre de Bruxelles, 1050 Brussels (BE); Centre National de la Recherche Scientifique, 75016 Paris (FR); Institut National de la Santé et de la Recherche Médicale, 75013 Paris (FR); Centre Hospitalier Régional de Grenoble, 38700 La Tronche (FR); COMMISSARIAT À L'ÉNERGIE ATOMIQUE ET AUX ÉNERGIES ALTERNATIVES, 75015 Paris (FR)
(72) Inventor: BUSSER, Benoît, 38700 LE SAPPEY-EN-CHARTREUSE (FR); DENIAUD, Aurélien, 38210 POLIENAS (FR); LELIEVRE, Pierre, 38190 LES ADRETS (FR); SANCEY GALLIOT, Lucie, 38850 VILLAGES DU LAC DE PALADRU (FR); RENIER, Nathan, 1410 Waterloo (BE); VALKENIER-VAN DIJK, Elisabeth H., 1170 Watermael-Boitsfort (BE); JABIN, Ivan, 1180 Uccle (BE)
(74) Representative: Germain Maureau

(57) **Abstract**

The present invention relates to calix[4]arenes of the formula (1), 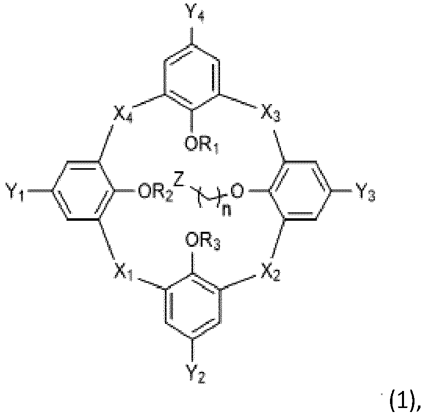 in which:
Y₁, Y₂, Y₃ and Y₄ are selected from the group consisting of hydrogen, NO₂, amine, azide, halogen, triazole and C₁-C₈-alkyl group, X₁, X₂, X₃ and X₄ are selected from the group consisting of CH₂ and S, Z is a heterocyclic moiety which is selected from the group consisting of imidazole, benzimidazole, benzothiazole, benzoxazole, purine, oxazole, triazole, pyrazole and thiazole, R₁, R₂ and R₃ are selected from the group consisting -(CH₂)ₙZ, hydrogen and C₁-C₈-alkyl group, n being an integer comprised between 1 and 4. They exhibit high cytotoxicity against cancer cells.

## Description

The present invention relates to the field of anticancer therapy. It particularly relates to calix[4]arenes which exhibit anticancer activity.

Cancers are one of the main causes of mortality. At the present time, one third of new cases of cancer exhibit resistance to multiple drugs or are chemoresistant. More precisely some compounds used for treating cancers may cause a development of chemoresistant tumor cells and/or cause secondary effects. Thus, there remains a need for finding novel compounds with improved properties for treating cancer.

Copper (hereafter abbreviated "Cu") is an essential element for human life. It plays an essential role in a large number of cellular mechanisms and signaling pathways. For example, Cu ions are involved in metabolic processes such as cellular respiration and a lot of enzymes use Cu in their active site, taking advantage of its redox and catalytic properties. Therefore, the intake of Cu and its transport across cellular membranes are essential. Specific membrane proteins, such as Ctr1, transport Cu across lipid bilayers as Cu⁺. Furthermore, dysregulation of Cu⁺ transport due to mutations in the genes encoding for such proteins leads to disruption of Cu homeostasis and causes serious health problems. The involvement of Cu in oxidation-reduction reactions requires close regulation of Cu metabolism in order to avoid toxic effects. Indeed, it has been shown that disturbances in Cu homeostasis lead to structural abnormalities or loss of certain essential physiological functions.

Regarding cancer, the increase of the Cu content in some cancer cells has been shown. In particular, variations in Cu protein levels have been demonstrated in many types of cancers. These variations result in abnormal concentrations of Cu in tumoral cells and alterations in the systemic distribution of Cu. Such alterations in Cu homeostasis may promote tumor growth or invasiveness or may even confer resistance to treatments (for example chemotherapy). More precisely, Cu is acting on different molecular pathways leading to a proangiogenic response necessary for carcinogenesis processes. It appears that Cu also influences the spread and formation of secondary tumors via the activation of enzymes responsible for cell proliferation.

The altered Cu metabolism in cancer cells and the different responses of tumor cells to Cu support the development of treatments that disrupt, deplete, or increase Cu levels in tumor cells. Indeed, the metallic nature of Cu is a key strategy for the development of anticancer compounds via the synthesis of Cu-based complexes.

In particular, some therapeutic strategies based on chelators or ionophores involving Cu have shown promising results in preclinical studies, and others at the clinical stage for the treatment of cancer. Indeed, the administration of Cu chelators and ionophores alter the concentration, distribution and redox behavior of Cu within the cancer cells.

Chelators directly bind and sequester the Cu cations in order to remove them or in other cases to induce stress in cancer cells, ultimately resulting in regulated cancer cell death (e.g. apoptosis or cuproptosis).

In contrast, ionophores cross cellular membranes in a reversibly Cu-bound form and release Cu on the other side of the membrane, generally leading to the increase of the intracellular concentrations of Cu cations. The bound Cu-ionophore complex is sufficiently lipophilic to facilitate membrane passage. Cu release can be triggered by a low local concentration of the Cu cation or the presence of a particular stimulus that promotes Cu cation release. The ability of certain ionophores to cause the death in cancer cells is well-known and has gained attention in recent years.

For example, elesclomol, disulfiram and clioquinol are known as useful ionophores for treating cancers. The clinical use of clioquinol has been discontinued because of its neurotoxicity. But this compound and its analogs are still tested in combination with different administration routes to maintain its anticancer effects while reducing toxicities. The anticancer efficacy of disulfiram was demonstrated in *in vitro* and *in vivo* models of inflammatory breast cancer. Moreover, the combination of disulfiram and docosahexaenoic acid has been shown to promote the death of cancer cells and to reduce the growth of cancer cells *in vitro* and *in vivo.*

Thus, the development of novel compounds having the capacity to disrupt Cu homeostasis in cancer cells and their proliferation is an interesting and promising approach for cancer therapy. Furthermore, the combination of these compounds with standard chemotherapy, or targeted therapy, or immunotherapy will be an efficient strategy to kill cancer cells.

Surprisingly, the inventors have discovered novel calix[4]arenes which exhibit anticancer activity and represent some very promising solutions for treating cancer.

More precisely, these novel calix[4]arenes have specific groups (in particular imidazole groups) that coordinate cations (for example Cu⁺, Cu²⁺, Ag⁺, Zn²⁺, preferably Cu⁺). Thus, they are able to bind cations (preferably Cu⁺). Furthermore, these calix[4]arenes are able to transport cations (preferably Cu⁺) across membranes and they have not a too high lipophilicity which allows them to be delivered to cancer cell membranes. Hereafter, the present invention is described in more detail in reference to the ability of these calix[4]arenes to coordinate the cation Cu⁺ without being limited to this aspect.

Moreover, a high cytotoxicity of the calix[4]arenes of the invention against different cancer cells types has been shown, at micromolar concentrations. It was observed that high cytotoxicity is linked to the high levels of Cu in cancer cells and the ability of these calix[4]arenes to transport Cu and thus to disrupt the Cu homeostasis in the cancer cells and their proliferation.

Thus, a subject of the present invention are calix[4]arenes of the formula (1), 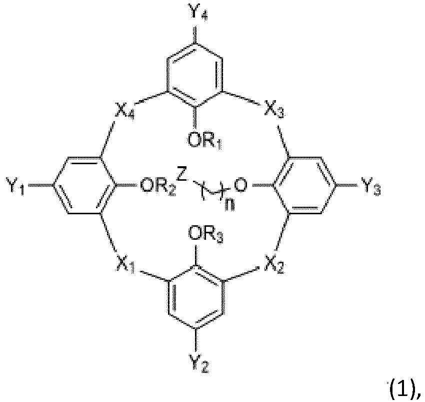
or physiologically tolerable salts thereof
in which:
   Y₁, Y₂, Y₃ and Y₄ are each independently selected from the group consisting of hydrogen, NO₂, amine, amide, azide, halogen, cyano, carboxylic acid ester, triazole and C₁-C₈-alkyl (preferably C₁-C₄-alkyl) group, said C₁-C₈-alkyl (preferably C₁-C₄-alkyl) group being optionally substituted with one or more substituents each independently selected from the group consisting of alkynyl, alkene, azide, ether, halogen, carboxylic acid ester, amide, cyano and triazole,
   with the proviso that no one of Y₁, Y₂, Y₃ and Y₄ are a *tert*-butyl group,
   X₁, X₂, X₃ and X₄ are each independently selected from the group consisting of CH₂ and S, Z is a heterocyclic moiety which is selected from the group consisting of imidazole, benzimidazole, benzothiazole, benzoxazole, purine, oxazole, triazole, pyrazole and thiazole, said heterocyclic moiety being optionally substituted with 1 to 5 substituents which are selected from the group consisting of hydrogen, halogen, alkyl, alkenyl, alkynyl, phenyl, benzyl, OH, ether, ester, amide, NO₂, amine, azide and nitrile,
   R₁, R₂ and R₃ are each independently selected from the group consisting of -(CH₂)ₙZ, hydrogen and C₁-C₈-alkyl (preferably C₁₋C₃-alkyl) group, said C₁-C₈-alkyl group (preferably C₁-C₃-alkyl group) being optionally substituted with one or more substituents each independently selected from the group consisting of carboxylic acid ester, alkenyl, alkynyl, phenyl, OH, azide, amide, halogen, SH and thioether group,
   n being an integer comprised between 1 and 4 (preferably equal to 1),
   with the proviso that when R₁ and R₃ are each hydrogen and n is equal to 1, Z is not a benzothiazole group.

"Halogen" is understood as meaning fluorine, chlorine, bromine, or iodine.

"Alkyl" includes both branched and linear saturated aliphatic hydrocarbon groups, having the specified number of carbon atoms. The term "C₁-Cₙ alkyl" means an alkyl group having from 1 to n carbon atoms.

"Alkenyl" is a branched or linear unsaturated hydrocarbon group having the specified number of carbon atoms and at least one carbon-carbon double bond without any carbon-carbon triple bond. For example, the term "C₂-alkenyl" designates an ethenyl group.

"Alkynyl" is branched or linear unsaturated hydrocarbon group having the specified number of carbon atoms and at least one carbon-carbon triple bond. For example, the term "C₂ alkynyl" designates an ethynyl group.

Y₁, Y₂, Y₃ and Y₄ may be identical or different. Preferably, they are identical. Preferably, they are each hydrogen.

In some embodiments of the invention, Y₂ and Y₄ are NO₂ and Y₁ and Y₃ are hydrogen.

X₁, X₂, X₃ and X₄ may be identical or different. Preferably, they are identical. Preferably, they are each CH₂.

In preferred embodiments, R₂ is -(CH₂)ₙZ.

In preferred embodiments, n is equal to 1.

If Z is an imidazole group, it is preferably a 1-C₁₋C₆-alkyl-imidazole group (and more preferably 1-methylimidazole, 1-ethylimidazole, 1-propylimidazole, 1-isopropylimidazole) or a 1-benzylimidazole group.

If Z is a purine group, it is preferably a 9-C₁₋C₆-alkyl-purine group (and more preferably 9-methylpurine, 9-ethylpurine, 9-propylpurine, 9-isopropylpurine) or a 9-benzylpurine group.

If Z is an oxazole group, it is preferably unsubstituted. In some embodiments, Z is an oxazole group which is substituted at the 2, 4, or 5 position with a halogen or C₁₋C₆-alkyl.

If Z is a thiazole group, it is preferably unsubstituted. In some embodiments, Z is a thiazole group which is substituted at the 2, 4, or 5 position with a halogen or C₁₋C₆-alkyl.

If Z is a benzimidazole group, it is preferably a 1-C₁₋C₆-alkyl-benzimidazole group (and more preferably 1-methylbenzimidazole, 1-ethylbenzimidazole, 1-propylbenzimidazole, 1-isopropylbenzimidazole) or a 1-benzylbenzimidazole group.

If Z is a triazole group, it is preferably a 1-C₁₋C₆-alkyl-triazole group (and more preferably 1-methyltriazole, 1-ethyltriazole, 1-propyltriazole, 1-isopropyltridazole) or a 1-benzyltriazole group.

If Z is a pyrazole group, it is preferably a 1-C₁₋C₆-alkyl-pyrazole group (and more preferably 1-methylpyrazole, 1-ethylpyrazole, 1-propylpyrazole, 1-isopropylpyrazole) or a 1-benzylpyrazole group.

If Z is an benzoxazole group, it is preferably unsubstituted. In some embodiments, Z is a benzoxazole group which is substituted at the 4, 5, 6, or 7 position with a halogen or C₁-C₆-alkyl.

If Z is a benzothiazole group, it is preferably unsubstituted. In some embodiments, Z is a benzothiazole group which is substituted at the 4, 5, 6, or 7 position with a halogen or C₁-C₆-alkyl.

In some embodiments, R₁ and R₃ are each ester groups, and preferably they are each ethyl acetate groups. In preferred embodiments of these embodiments, R₂ is -(CH₂)ₙZ with n an integer comprised between 1 and 4 (preferably equal to 1) and Z is a substituted or unsubstituted imidazole group, preferably 1-methylimidazole.

In preferred embodiments of the invention, X₁, X₂, X₃ and X₄ are each CH₂, Y₁, Y₂, Y₃ and Y₄ are each hydrogen, R₁ and R₃ are each selected from the group consisting of hydrogen, C₁-C₆-alkyl, propene and ethyl acetate group, R₂ is -(CH₂)ₙZ and Z is selected from the group consisting of 1-methylimidazole, 1-methylbenzimidazole, 9-methylpurine, oxazole and thiazole group with n being an integer comprised between 1 and 4 (preferably equal to 1). In preferred embodiments of these embodiments, Z is 1-methylimidazole with n being an integer comprised between 1 and 4 (preferably equal to 1).

Some preferred calix[4]arenes of the invention are the calix[4]arenes of the formulas (2), (3) and (4):

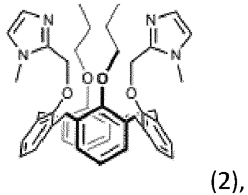

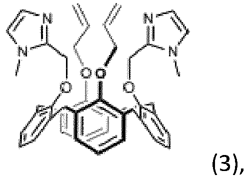

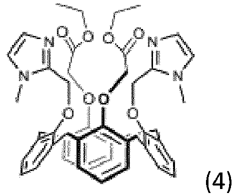

The most preferred calix[4]arene of the invention is the calix[4]arene of the formula (2).

Physiologically tolerable salts of the calix[4]arenes of the present invention are nontoxic salts that are physiologically acceptable, in particular pharmaceutically utilizable salts.

Salts of the calix[4]arenes of the present invention can be obtained by customary methods known to those skilled in the art, for example by combining a calix[4]arene of the present invention with an inorganic or organic salt in a solvent or diluent, or from other salts by cation exchange or anion exchange.

As explained above, the inventors have surprisingly discovered that the calix[4]arenes of the present invention as such have a high cytotoxicity against cancer cells thanks to their ability of disrupting the Cu homeostasis in the cancer cells and their proliferation. Moreover, the inventors have discovered that 1:1 complexes formed from these calix[4]arenes or physiologically tolerable salts thereof have a cytotoxicity against cancer cells too.

Thus, a further object of the invention are 1:1 complexes formed from a calix[4]arene of the invention or a physiologically tolerable salt thereof as described above and a cation, preferably Cu⁺.

The calix[4]arenes and the complexes as described above are prepared according to any conventional methods and known for those of skill in the art.

As a non-limiting example, the Cu⁺ complex of the calix[4]arene of the formula (2) is formed by mixing the calix[4]arene of the formula (2) with 1.5 equivalent of Cu⁺(MeCN)₄BF₄ in a mixture of CHCl₃ and MeCN, and the solvents are evaporated to obtain the salt of the formula (2a) :

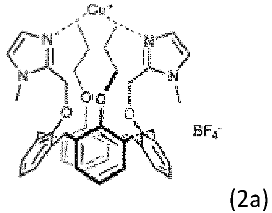

The calix[4]arenes, the physiologically tolerable salts thereof and the complexes of the present invention are valuable pharmacologically active elements. As explained above, they especially exhibit a cytotoxicity that is suitable, for example, for the treatment of cancer.

A further subject of the present invention is a pharmaceutical composition, which comprises at least one calix[4]arene of the formula (1) 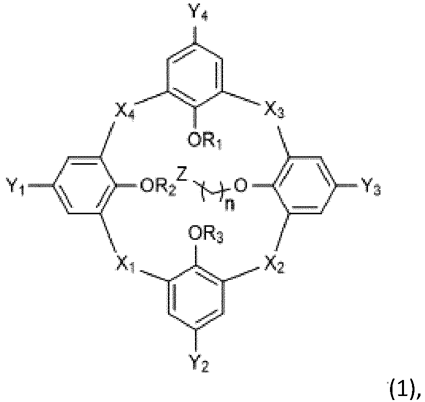 or a physiologically tolerable salt thereof or a 1:1 complex formed from a calix[4]arene of the formula (1) or from a physiologically tolerable salt thereof and a cation (preferably Cu⁺) in which:
Y₁, Y₂, Y₃ and Y₄ are each independently selected from the group consisting of hydrogen, NO₂, amine, amide, azide, halogen, cyano, carboxylic acid ester, triazole and C₁-C₈-alkyl (preferably C₁-C₄-alkyl) group, said C₁-C₈-alkyl (preferably C₁-C₄-alkyl) group being optionally substituted with one or more substituents each independently selected from the group consisting of alkynyl, alkene, azide, ether, halogen, carboxylic acid ester, amide, cyano and triazole,
with the proviso that no one of Y₁, Y₂, Y₃ and Y₄ are a *tert*-butyl group,
X₁, X₂, X₃ and X₄ are each independently selected from the group consisting of CH₂ and S, Z is a heterocyclic moiety which is selected from the group consisting of imidazole, benzimidazole, benzothiazole, benzoxazole, pyridine, purine, oxazole, pyrazole, thiazole, triazole, pyrimidine, pyrazine and pyridazine, said heterocyclic moiety being optionally substituted with 1 to 5 substituents which are selected from the group consisting of hydrogen, halogen, alkyl, alkenyl, alkynyl, phenyl, benzyl, imidazole, benzimidazole, benzothiazole, benzoxazole, pyridine, purine, oxazole, pyrazole, thiazole, triazole, pyrimidine, pyrazine, pyridazine, OH, SH, thioether, ether, ester, amide, NO₂, amine, azide and nitrile,
R₁, R₂ and R₃ are each independently selected from the group consisting of -(CH₂)ₙZ, hydrogen and C₁-C₈-alkyl (preferably C₁₋C₃-alkyl) group, said C₁-C₈-alkyl group (preferably C₁-C₃-alkyl group) being optionally substituted with one or more substituents each independently selected from the group consisting of carboxylic acid ester, alkenyl, alkynyl, phenyl, OH, azide, cyano, amide, halogen, SH and thioether group,
n being an integer comprised between 1 and 8 (preferably equal to 1),
and together with at least one pharmaceutically acceptable carrier.

Y₁, Y₂, Y₃ and Y₄ may be identical or different. Preferably, they are identical. Preferably, they are each hydrogen.

In some embodiments of the invention, Y₂ and Y₄ are NO₂ and Y₁ and Y₃ are hydrogen.

X₁, X₂, X₃ and X₄ may be identical or different. Preferably, they are identical. Preferably, they are each CH₂.

In preferred embodiments, R₂ is -(CH₂)ₙZ.

In preferred embodiments, n is equal to 1.

If Z is an imidazole group, it is preferably a 1-C₁₋C₆-alkyl-imidazole group (and more preferably 1-methylimidazole, 1-ethylimidazole, 1-propylimidazole, 1-isopropylimidazole) or a 1-benzylimidazole group.

If Z is a purine group, it is preferably a 9-C₁₋C₆-alkyl-purine group (and more preferably 9-methylpurine, 9-ethylpurine, 9-propylpurine, 9-isopropylpurine) or a 9-benzylpurine group.

If Z is an oxazole group, it is preferably unsubstituted. In some embodiments, Z is an oxazole group which is substituted at the 2, 4, or 5 position with a halogen or C₁₋C₆-alkyl.

If Z is a thiazole group, it is preferably unsubstituted. In some embodiments, Z is a thiazole group which is substituted at the 2, 4, or 5 position with a halogen or C₁₋C₆-alkyl.

If Z is an benzoxazole group, it is preferably unsubstituted. In some embodiments, Z is a benzoxazole group which is substituted at the 4, 5, 6, or 7 position with a halogen or C₁-C₆-alkyl.

If Z is a benzothiazole group, it is preferably unsubstituted. In some embodiments, Z is a benzothiazole group which is substituted at the 4, 5, 6, or 7 position with a halogen or C₁-C₆-alkyl.

If Z is a benzimidazole group, it is preferably a 1-C₁₋C₆-alkyl-benzimidazole group (and more preferably 1-methylbenzimidazole, 1-ethylbenzimidazole, 1-propylbenzimidazole, 1-isopropylbenzimidazole) or a 1-benzylbenzimidazole group.

If Z is a triazole group, it is preferably a 1-C₁₋C₆-alkyl-triazole group (and more preferably 1-methyltriazole, 1-ethyltriazole, 1-propyltriazole, 1-isopropyltridazole) or a 1-benzyltriazole group.

If Z is a pyrazole group, it is preferably a 1-C₁₋C₆-alkyl-pyrazole group (and more preferably 1-methylpyrazole, 1-ethylpyrazole, 1-propylpyrazole, 1-isopropylpyrazole) or a 1-benzylpyrazole group.

If Z is a pyridine group, it is preferably unsubstituted. In some embodiments, Z is a pyridine group which is substituted at the 2, 3, 4, 5, or 6 position with a halogen, C₁-C₆-alkyl, alkenyl, alkynyl, phenyl, benzyl, OH, SH, thioether, ether, ester, amide, NO₂, amine, azido or nitrile group.

If Z is a pyrimidine group, it is preferably unsubstituted. In some embodiments, Z is a pyrimidine group which is substituted at the 2, 4, 5, or 6 position with a halogen, C₁-C₆-alkyl, alkenyl, alkynyl, phenyl, benzyl, OH, SH, thioether, ether, ester, amide, NO₂, amine, azido or nitrile group.

If Z is a pyrazine group, it is preferably unsubstituted. In some embodiments, Z is a pyrazine group which is substituted at the 2, 3, 5, or 6 position with a halogen, C₁-C₆-alkyl, alkenyl, alkynyl, phenyl, benzyl, OH, SH, thioether, ether, ester, amide, NO₂, amine, azido or nitrile group.

If Z is a pyridazine group, it is preferably unsubstituted. In some embodiments, Z is a pyridazine group which is substituted at the 3, 4, 5, or 6 position with a halogen, C₁-C₆-alkyl, alkenyl, alkynyl, phenyl, benzyl, OH, SH, thioether, ether, ester, amide, NO₂, amine, azido or nitrile group.

In some embodiments, R₁ and R₃ are each ester groups, and preferably they are each ethyl acetate groups. In preferred embodiments of these embodiments, R₂ is -(CH₂)ₙZ with n an integer comprised between 1 and 4 (preferably equal to 1) and Z is a substituted or unsubstituted imidazole group, preferably 1-methylimidazole.

In preferred embodiments of the invention, X₁, X₂, X₃ and X₄ are each CH₂, Y₁, Y₂, Y₃ and Y₄ are each hydrogen, R₁ and R₃ are each selected from the group consisting of hydrogen, C₁-C₆-alkyl, propene and ethyl acetate group, R₂ is -(CH₂)ₙZ and Z is selected from the group consisting of 1-methylimidazole, 1-methylbenzimidazole, 9-methylpurine, oxazole and thiazole group with n being an integer comprised between 1 and 4 (preferably equal to 1). In preferred embodiments of these embodiments, Z is a 1-methylimidazole with n being an integer comprised between 1 and 4 (preferably equal to 1).

In preferred embodiments, the pharmaceutical composition comprises at least one calix[4]arene of the formulas (2), (3) or (4):

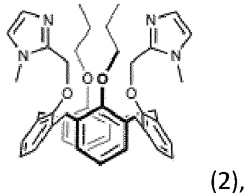

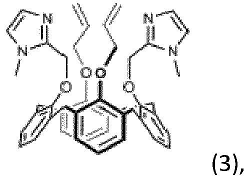

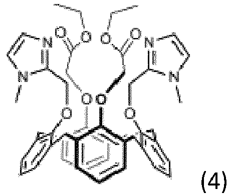

More preferably, the pharmaceutical composition comprises the calix[4]arene of the formula (2).

"Carrier" means solvent, dispersion medium, diluent, excipient, or vehicle with which at least one compound of the present invention is administered. A "pharmaceutically acceptable carrier" means a substance, e.g., excipient, diluent, or vehicle, which is useful in preparing the pharmaceutical composition that is safe, non-toxic and neither biologically nor otherwise undesirable, and includes a carrier that is acceptable for veterinary use as well as human pharmaceutical use. A "pharmaceutically acceptable carrier" includes both one and more than one such carrier.

Carriers include excipients and diluents and must be of sufficiently high purity and sufficiently low toxicity to render them suitable for administration to the patient being treated. The carrier can be inert or it can possess pharmaceutical benefits of its own. The amount of carrier employed in conjunction with the compound is sufficient to provide a practical quantity of material for administration per unit dose of the compound.

Classes of carriers include, but are not limited to binders, buffering agents, coloring agents, diluents, disintegrants, emulsifiers, flavorings, glidants, lubricants, preservatives, stabilizers, surfactants, tableting agents, and wetting agents. Some carriers may be listed in more than one class, for example vegetable oil may be used as a lubricant in some formulations and a diluent in others. Exemplary pharmaceutically acceptable carriers include sugars, starches, celluloses, powdered tragacanth, malt, gelatin, talc, and vegetable oils. Optional active and/or inactive agents may be included in the pharmaceutical compositions, provided that such agents do not substantially interfere with the activity of the compounds of the present invention. The optional active agent is an additional active agent that is not a compound or a salt or a complex of the present invention.

In the pharmaceutical composition, the calix[4]arene as described above or the physiologically tolerable salt thereof or the complex as described above may be in any appropriate formulation aiming at improving/modifying its solubility, its features of targeting, improving its (bio)availability, stability, toxicity, potency, physiochemical/metabolic properties, pharmacokinetics properties, disposition in an organism.

Preferably, in the pharmaceutical composition, the calix[4]arene as described above or the physiologically tolerable salt thereof or the complex as described above complies with the LADMET criteria. "LADMET" is an acronym for "liberation, absorption, distribution, metabolism, excretion and toxicity".

In preferred embodiments of the pharmaceutical composition, because of its high lipophilicity, the calix[4]arene as described above or the physiologically tolerable salt thereof or the complex as described above is formulated in the form of a lipidic formulation (more preferably nanoformulation), lipoproteins such as low density lipoprotein ("LDL") or Very Low Density Lipoprotein ("VLDL") or integrated in natural or synthetic membranes such as erythrocytes, engineered cells or vesicles.

In preferred embodiments of the invention, the pharmaceutical composition further comprises a least one compound selected from the group consisting of chemotherapy compounds (i.e. cancer chemotherapeutic agents), immunotherapy compounds, antiviral compounds and antibiotics.

In most preferred embodiments of the invention, the pharmaceutical composition comprises:
- at least one calix[4]arene as described above or a physiologically tolerable salt thereof or a complex as described above and
- at least one anticancer compound.

In other words, in most preferred embodiments of the invention, the calix[4]arene is used in combination with other anticancer compounds.

In preferred embodiments of the invention, the pharmaceutical composition comprises:
- at least one calix[4]arene as described above or a physiologically tolerable salt thereof or a complex as described above and
- at least one compound selected from the group consisting of Cu salts (encapsulated (for example encapsulated in liposomes) or not), copper or copper oxide nanoparticles and Cu chelates.

In other words, in preferred embodiments of the invention, the calix[4]arene of the present invention is used in combination with Cu salts (encapsulated or not), copper or copper oxide nanoparticles, Cu chelates. Indeed, the inventors discovered that these combinations boost the cytotoxicity of the calix[4]arenes of the invention.

In most preferred embodiments of the invention, the pharmaceutical composition comprises:
- at least one calix[4]arene as described above or a physiologically tolerable salt thereof or a complex as described above and
- CuCl₂ or CuSO₄, or any other physiologically compatible Cu(ll) molecule/salt.

The Cu salt is preferably CuCl₂.

In embodiments of the invention, the calix[4]arene as described above or the physiologically tolerable salt thereof or the complex as described above may be, for example, administrated by intravenous injection and the Cu salts or the copper or copper oxide nanoparticles may be administrated thanks to a patch.

The pharmaceutical composition of the present invention may be administered orally, topically, parenterally (intravenous, intradermal, subcutaneous, intramuscular), intratumorally, intraarticularly, by inhalation or spray, sublingually, transdermally, rectally, as an ophthalmic solution, or by other means, in dosage unit formulations containing conventional pharmaceutically acceptable carriers.

The pharmaceutical composition may be formulated as any pharmaceutically useful form, e.g., as an aerosol, a cream, a gel, a pill, a capsule, a tablet, a syrup, a transdermal patch, or an ophthalmic solution. Some dosage forms, such as tablets and capsules, are subdivided into suitably sized unit doses containing appropriate quantities of the active components, e.g., an effective amount to achieve the desired purpose.

The pharmaceutical composition is preferably formulated for intravenous administration.

The pharmaceutical compositions may contain between 0.001 % and 99 % by weight of a calix[4]arene as described above or a physiologically tolerable salt thereof or a complex as described above.

A further subject of the present invention is a calix[4]arene as described above or a physiologically tolerable salt thereof or a complex as described above for use as a medicament.

A further subject of the present invention is a calix[4]arene as described above or a physiologically tolerable salt thereof or a complex as described above for use in the treatment of a disease selected from the group consisting of cancer or metastasis thereof, viral, bacterial, parasitic and fungal infections.

The cancer may be any kind of solid or haematological malignancy, notably those selected from the group consisting of oral cancer, oropharyngeal cancer, nasopharyngeal cancer, respiratory cancer, urogenital cancer, gastrointestinal cancer, central or peripheral nervous system tissue cancer, an endocrine or neuroendocrine cancer or hematopoietic cancer, glioma, sarcoma, carcinoma, lymphoma, melanoma, fibroma, meningioma, brain cancer, oropharyngeal cancer, nasopharyngeal cancer, renal cancer, biliary cancer, pheochromocytoma, pancreatic islet cell cancer, Li-Fraumeni tumors, thyroid cancer, parathyroid cancer, pituitary tumors, adrenal gland tumors, osteogenic sarcoma tumors, multiple neuroendocrine type I and type II tumors, breast cancer, lung cancer, head and neck cancer, prostate cancer, esophageal cancer, tracheal cancer, liver cancer, bladder cancer, stomach cancer, pancreatic cancer, ovarian cancer, uterine cancer, cervical cancer, testicular cancer, colon cancer, rectal cancer and skin cancer. In some aspects, the patient has an epithelial cancer. In yet further aspects, the patient has an endometrial cancer, an ovarian cancer or a melanoma. In some aspects, the patient has hematological malignancies such as B-cell lymphoid neoplasm, T-cell lymphoid neoplasm, non-Hodgkin lymphoma (NHL), B-NHL, T-NHL, chronic lymphocytic leukemia (CLL), small lymphocytic lymphoma (SLL), mantle cell lymphoma (MCL), NK-cell lymphoid neoplasm and myeloid cell lineage neoplasm including acute myeloid leukemia. In further aspects, the patient is a patient that has previously received one or more anti-cancer therapy or has pervious failed to adequately respond to one or more anti-cancer therapy. Thus, in some aspects, the cancer is a cancer that is resistant to at least a first anti-cancer therapy.

The bacterial infection may be any bacterial infection due to Gram + or Gram - bacteria and may be, for example, selected from the group consisting of pneumonia, bacterial meningitis, cholera, diphtheria, tuberculosis, anthrax, botulism, brucellosis, campylobacteriosis, typhus, gonorrhea, listeriosis, lyme disease, rheumatic fever, pertussis (Whooping Cough), plague, salmonellosis, scarlet fever, shigellosis, syphilis, tetanus, trachoma, tularemia, typhoid fever and urinary tract infections.

The parasitic infection may be selected from the group consisting of malaria, leishmaniasis, trypanosomiasis, chagas disease, cryptosporidiosis, fascioliasis, filariasis, amebic infections, giardiasis, pinworm infection, schistosomiasis, taeniasis, toxoplasmosis, trichinellosis and trypanosomiasis.

The fungal infection may be selected from the group consisting of candidiasis, aspergillosis, coccidioidomycosis, cryptococcosis, histoplasmosis and tinea pedis.

A further subject of the present invention is a calix[4]arene as described above or a physiologically tolerable salt thereof or a complex as described above for use against yeasts, fungi, bacteria, viruses, or other microorganisms in or on living man or living animal.

The present invention is illustrated below with the aid of examples.

Brief description of the figures:
Figure 1 is a graph showing the profile of the proliferation index Nₜ/N₀ of the A549 cells after exposure or not with the calix[4]arene of the formula (2).
Figure 2 is a graph showing the profile of the proliferation index Nₜ/N₀ of the PC9 cells after exposure or not with the calix[4]arene of the formula (2).
Figure 3 is a graph showing the profile of the proliferation index Nₜ/N₀ of the H322 cells after exposure or not with the calix[4]arene of the formula (2).

### EXAMPLES:

### I - DESCRIPTION AND PREPARATION OF CALIX[4]ARENES OF THE PRESENT INVENTION AND TWO COMPARATIVE EXAMPLES:

### Example 1: Synthesis and characterization of the calix[4]arene of the formula (2) :

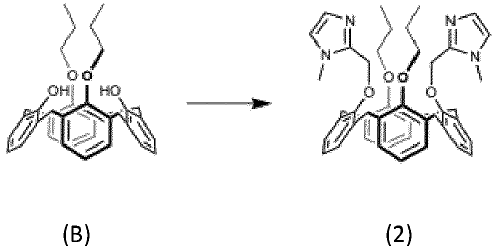

In a 50 mL round bottom flask, the calix[4]arene of the formula (B) (309 mg, 0.61 mmol, 1.0 equiv.) was stirred in anhydrous THF (12 mL) with NaH (60% dispersion in mineral oil, 520 mg, 13.0 mmol, 36 equivalents) for 30 minutes under inert atmosphere. 2-(chloromethyl)-1-methyl-1H-imidazole hydrochloride (407 mg, 3.11 mmol, 5.1 equiv.) dissolved in anhydrous DMF (3 mL) was then added. The mixture was stirred at reflux for 24 hours under inert atmosphere. After reaction, the mixture was brought to room temperature and the solvent was evaporated under reduced pressure. The residue was dissolved in DCM (20 mL) and then washed with water (3x10 mL). The organic layer was evaporated under reduced pressure. The crude product was purified by flash chromatography (DCM/MeOH 95:5 v/v). The resulting product was washed with H₂O affording the calix[4]arene of the formula (2) (269 mg, 0.38 mmol) as a white solid. Yield 64%.

¹H NMR (400 MHz, CDCl₃): δ_{H} (ppm) = 7.04 (s, 2H, Im**H**), 6.88-6.94 (m, 6H, Ar**H** and Im**H**), 6.80 (bt, 2H, Ar**H**), 6.33 (bt, 2H, Ar**H**), 6.24 (s, 4H, Ar**H**), 4.92 (s, 4H, OC**H**₂Im), 4.34 (d, *J* = 13 Hz, 4H, ArC**H**ₐₓ), 3.81 (t, *J* = 8 Hz, 4H, OC**H**₂Et), 3.50 (s, 6H, NC**H**₃), 3.04 (d, *J* = 13 Hz, 4H, ArC**H**_{eq}), 1.71-1.77 (m, 4H, C**H**₂), 0.75 (t, *J* = 8 Hz, 6H, C**H**₃); HRMS (ESI): calculated for C₄₄H₄₉N₄O₄⁺[M+H⁺]: 696.3676, measured: 696.3674.

### Example 1a: Synthesis and characterization of the calix[4]arene CuBF₄ complex of the formula (2a):

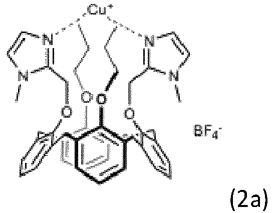

The Cu⁺ complex of the calix[4]arene of the formula (2) was formed by mixing (2) with 1.5 equivalent of Cu⁺(MeCN)₄BF₄ in a mixture of CHCl₃ and MeCN, and the solvents were evaporated to obtain the salt of the formula (2a).

¹H NMR (400 MHz, CDCl_{3/}CD₃CN 1/4, 298 K): δ (ppm) = 7.41 (s, Im**H**, 2H), 7.22 (bd, Ar**H**, 4H), 7.11 (s, Im**H**, 2H), 7.09 (bt, Ar**H**, 2H), 6.20 (bt, Ar**H**, 2H), 6.03 (bd, Ar**H**, 4H), 5.33 (s, OC**H**₂Im, 4H), 3.78 (bd, ArC**H**₂, 4H), 3.70 (t, ³*J* = 7.6 Hz, OC**H**₂Et, 4H), 3.10 (bd, *J* = 11.6 Hz, ArC**H**₂, 4H), 2.85 (s, NC**H**₃, 6H), 1.75-1.86 (m, C**H**₂, 4H), 0.96 (t, *J* = 7.6 Hz, C**H**₃, 6H).

### Example 2: Synthesis and characterization of the calix[4]arene of the formula (5)

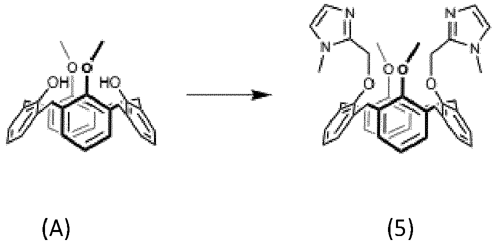

In a 100 mL RBF was added the calix[4]arene of the formula (A) (1.0 g, 2.21 mmol, 1 equivalent) and the reagent was dried under high vacuum for 1 hour, then purged under argon and dissolved in 25 mL of freshly distilled THF. The mixture was cooled to 0°C and then NaH (washed 3 times with petroleum ether and then dried under high vacuum, 1.11 g, 77.3 mmol, 35 equivalents) was added. The reaction mixture was stirred at this temperature for 15 minutes then 2-(chloromethyl)-1-methyl-1H-imidazole hydrochloride (dried under high vacuum, 1.84 g, 11.05 mmol, 5 equivalents) was added in one portion. The reaction was refluxed overnight and monitored by ¹H NMR. Afterwards, the reaction was cooled to room temperature and the volatiles were removed under reduced pressure. 50 mL of dichloromethane were added followed by 50 mL of distilled water and the product was extracted with additional 2×50 mL of dichloromethane. The organic layers were combined and the volatiles were removed under reduced pressure. The resulting crude was then purified by column chromatography on silica (DCM/MeOH:98/2) to afford the calix[4]arene of the formula (5) as a white solid in 35% yield (500 mg, 0.77 mmol).

HRMS (ESI): calculated for C₄₀H₄₁N₄O₄⁺[M+H⁺]: 641.3122, measured: 641.3133 [M+H]⁺ and 663.2951 [M+Na]⁺. The ¹H NMR spectrum of the calix[4]arene of the formula (5) in CDCl₃ has broad features, thus 1 eq. of Cu⁺(MeCN)₄BF₄ was added to characterise the Cu⁺ complex of the calix[4]arene of the formula (5). ¹H NMR (CDCl_{3/}CD₃CN 4/1, 400 MHz) δ_{H} (ppm) = 7.44 (s, 2H, Im**H**), (bd, J= 7.2 Hz, 4H, Ar**H**), 7.13 (s, 2H, Im**H**), 7.09 (bt, J = 7.2 Hz, 2H, Ar**H**), 6.26 (bt, J = 6.8 Hz, 2H, ArH), 6.16 (bd, ²J = 7.2 Hz, 4H, ArH), 5.41 (s, OC**H**₂, 4H), 3.8-3.57 (m, 10H, ArC**H**₂ and NC**H**₃), 3.12 (bd, J = 12.8 Hz, 4H, ArC**H**₂), 2.88 (s, 6H, C**H**₃).

### Example 3: Synthesis and characterization of the calix[4]arene of the formula (3):

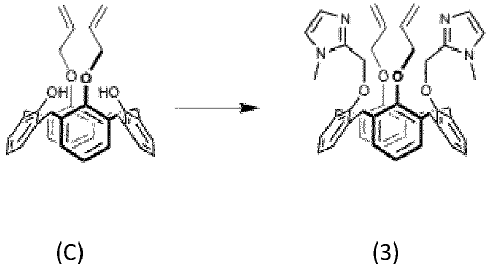

In a 100 mL RBF was added the calix[4]arene of the formula (C) (0.200 g, 0.40 mmol, 1 eq.) and the compound was dried under high vacuum for 1 hour, then purged under argon and dissolved in 25 mL of freshly distilled THF. The mixture was cooled to 0°C and then NaH (washed 3 times with petroleum ether and then dried under high vacuum, 0.1427 g, 9.9 mmol, 25 equivalents) was added. The reaction mixture was stirred at this temperature for 15 minutes then 2-(chloromethyl)-1-methyl-1H-imidazole hydrochloride (dried under high vacuum, 0.3310 g, 1.98 mmol, 5 equivalents) was added in one portion. The reaction was refluxed overnight and monitored by ¹H NMR. Afterwards, the reaction was cooled to room temperature and the volatiles were removed under reduced pressure. 50 mL of dichloromethane were added followed by 50 mL of distilled water. The product was extracted with additional 2×50 mL of dichloromethane. The organic layers were collected and the volatiles were removed under reduced pressure. The resulting brownish oil was purified by column chromatography on silica (DCM to DCM/MeOH 98/2) to afford the calix[4]arene of the formula (3) as a white solid with 70% yield (0.1922 g, 0.30 mmol).

¹H NMR (CDCl₃, 300 MHz) δ_{H} (ppm) = 7.06-7.04 (m, Ar**H** and Im**H**, 6H), 6.94-6.84 (m, Ar**H** and Im**H**, 4H), 6.32 to 6.14 (m, Ar**H** and CH₂-C**H**=, 8H), 5.02-4.90 (m, =C**H**₂, 4H), 4.88 (s, OC**H**₂, 4H), 4.49 (d, *J* = 6.7 Hz, C**H**₂-CH=, 4H), 4.33 (d, *J* = 13.4 Hz, ArC**H**ₐₓ, 4H), 3.49 (s, NC**H**₃, 6H), 3.02 (d, *J* = 13.5 Hz, ArC**H**_{eq}, 4H); HRMS (ESI): calculated for C₄₄H₄₅N₄O₄⁺[M+H⁺]: 693.3435, measured: 693.3448 [M+H]⁺ and 715.3276 [M+Na]⁺.

### Example 4: Synthesis and characterization of the calix[4]arene of the formula (6):

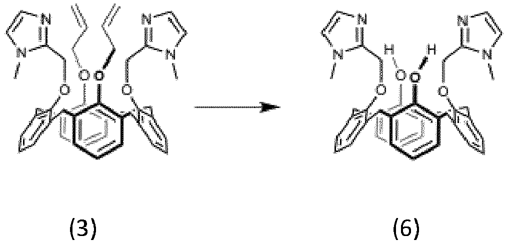

In a RBF were added the calix[4]arene of the formula (3) (0.400g, 0.58 mmol), Pd(OAc)₂ (0.0194 g, 0.09 mmol, 0.15 equivalents) and PPh₃ (0.0454g, 0.173 mmol, 0.3 equivalents) with 25 mL of freshly distilled THF and 5 mL of distilled water. NEt₂H (3.5 mL, 1.478 g, 20.2 mmol, 35 equivalents) was added and the reaction was refluxed under argon overnight. The conversion was monitored by ¹H NMR and then stopped upon completion of the reaction. The mixture was cooled to room temperature, filtered through celite followed by washing with dichloromethane 3×30 mL. The combined organic fractions were washed with 3×25 mL of distilled water and the volatiles were removed under reduced pressure. Afterwards, the resulting oil was purified by column chromatography on silica (DCM to DCM/MeOH 95/5) to afford the calix[4]arene of the formula (6) as a pinkish solid with 60% yield (0.213 g, 0.35 mmol).

¹H NMR (CDCl₃, 300 MHz) δ_{H} (ppm) = 7.49 (s, O**H**, 2H), 7.11-7.01 (m, Ar**H** and Im**H**, 6H), 6.98 (s, Im**H**, 2H), 6.87 (d, *J* = 7.4 Hz, Ar**H**, 4H), 6.79 to 6.62 (m, Ar**H**, 4H), 5.05 (s, OC**H**₂, 4H), 4.22 (d, *J* = 13.1 Hz, ArC**H**ₐₓ, 4H), 3.77 (s, NC**H**₃, 4H), 3.37 (d, *J* = 13.1 Hz, ArC**H**_{eq}, 4H); HRMS (ESI): calculated for C₃₈H₃₇N₄O₄⁺[M+H⁺]:613.2809, measured: 613.2833 [M+H]⁺and 635.2654 [M+Na]⁺.

### Example 5: Synthesis and characterization of the calix[4]arene of the formula (7):

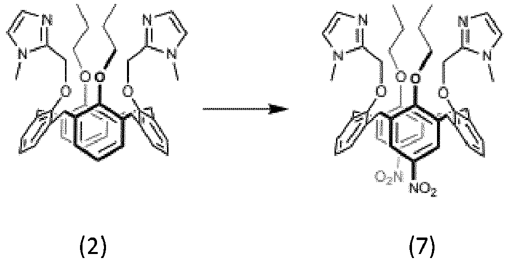

The calix[4]arene of the formula (2) (0.050 g, 0.072 mmol, 1 equivalent) was dissolved in CH₂Cl₂ (2.5 mL). A mixture of glacial CH₃COOH/fuming HNO₃ (1:1, 0.54 mL) was added at 0°C and the reaction mixture was stirred for 25 hours at room temperature. The reaction mixture was concentrated under reduced pressure. The crude residue was dissolved in CH₂Cl₂ and washed with H₂O until pH6. The solvent was then evaporated under reduced pressure to yield the calix[4]arene of the formula (7) (0.042 g, 74%) as a solid.

¹H NMR (400 MHz, CDCl_{3/}CD₃CN 1/4, 298 K): δ (ppm) = 7.53 (s, Im**H**, 2H), 7.3-7.38 (m, Ar**H**, 4H), 7.16-7.25 (m, Ar**H** and Im**H**, 4H), 6.88 (s, Ar**H**, 4H), 5.31 (s, OCH₂Im,4H), 3.91 (bd, *J* = 14 Hz, ArC**H**₂, 4H), 3.67 (t, ³*J* = 7.2 Hz, OC**H**₂Et, 4H), 3.28 (d, *J* = 14 Hz, ArC**H**₂, 4H), 3.16 (s, NC**H**₃, 6H), 1.61-1.72 (m, C**H**₂, 4H), 0.90 (t, ³*J* = 7.2 Hz, C**H**₃, 6H).

### Example 6: Synthesis and characterization of the calix[4]arene of the formula (8):

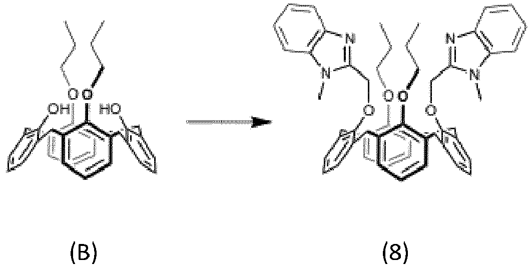

In a 50 mL RBF was added the calix[4]arene of the formula (B) (0.200 g, 0.39 mmol), dried under high vacuum and then purged under argon, with 25 mL of freshly distilled THF. The solution was cooled to 0°C and then NaH (freshly washed with 2×15 mL of petroleum ether, dried under high vacuum and then purged under argon, 0.236 g, 9.8 mmol, 25 eq.) was added to the solution. The reaction mixture was stirred at 0°C for 30 minutes and then 2-(chloromethyl)-1-methyl-1H-benzimidazole (0.213 g, 1.18 mmol, 3 eq.) dried and purged under argon, was added. The reaction mixture was heated to room temperature and then refluxed overnight under argon. Afterwards, the reaction was cooled to room temperature, quenched with slow addition of 30 mL of distilled water into the solution and then the product was extracted with 3×50 mL of dichloromethane. The organic layers were combined then the volatiles were removed under reduced pressure. The product was purified by column chromatography on silica (DCM to DCM/MeOH 98/2) and the volatiles were removed under reduced pressure. The product was dissolved in a minimum amount of chloroform and then precipitated by slow addition of 100 mL of pentane, to afford the calix[4]arene of the formula (8) as a purple solid with 59% yield (0.184 g, 0.23 mmol).

¹H NMR (CDCl₃, 300 MHz) δ_{H} (ppm) = 7.78 (d, *J* = 6.7 Hz, Bzlm**H**, 2H), 7.35-7.21 (m, Bzlm**H**, 6H), 6.93 (d, *J* = 7.3 Hz, Ar**H**, 4H), 6.80 (t, *J* = 7.4 Hz, Ar**H**, 2H), 6.35 (t, *J* = 7.3 Hz, Ar**H**, 2H), 6.25 (d, *J* = 7.4 Hz, Ar**H**, 4H), 5.14 (s, OC**H**₂, 4H), 4.41 (d, *J* = 13.4 Hz, ArC**H**ₐₓ, 4H), 3.76 to 3.70 (m, NC**H**₃ + OC**H**₂, 10H), 3.09 (d, *J* = 13.4 Hz, ArC**H**_{eq}, 4H), 1.59 (sext, *J* = 8.3 Hz, C**H**₂CH₃, 4H), 0.44 (t, *J* = 7.4 Hz, C**H**₃, 6H); HRMS (ESI): calculated for C₅₂H₅₃N₆O₆⁺ [M+H⁺]: 797.4071 measured: 797.4070( [M+H]⁺ and 819.3891 [M+Na]⁺.

### Example 7: Synthesis and characterization of the calix[4]arene of the formula (4):

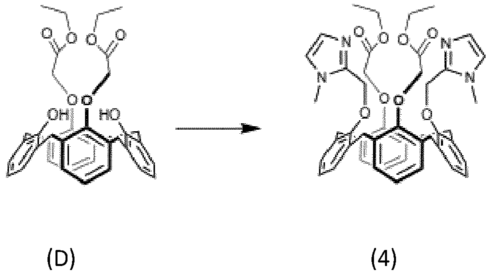

In a 100 mL RBF was added the calix[4]arene of the formula (D) (0.500 g, 0.84 mmol, 1 eq.), 2-(chloromethyl)-1-methyl-1H-imidazole hydrochloride (dried under high vacuum, 0.700 g, 4.19 mmol) and the mixture was dried under high vacuum for 1 hour, then purged under argon and dissolved in 25 mL of freshly distilled THF. The mixture was cooled to 0°C and then NaH (washed 3 times with petroleum ether and then dried under high vacuum, 0.503 g, 20.9 mmol, 25 equivalents) was added. The reaction was refluxed overnight and monitored by ¹H NMR. Afterwards, the reaction was cooled to room temperature and the volatiles were removed under reduced pressure. 50 mL of dichloromethane were added followed by 50 mL of distilled water. The product was extracted with additional 2×50 mL of dichloromethane. The organic layers were collected and the volatiles were removed under reduced pressure. The resulting brownish oil was purified by column chromatography on silica (DCM to DCM/MeOH 90/10) to afford the calix[4]arene of the formula (4) as a white solid (0.104 g, 0.13 mmol) with 15% yield.

¹H NMR (CDCl₃, 300 MHz) δ_{H} (ppm) = 7.03 (bs, Im**H**, 2H), 6.93 to 6.74 (m, Im**H** and Ar**H**,6 H), 6.36 (t, *J* = 7.2 Hz, Ar**H**, 2H), 6.26 (bd, *J* = 7.6 Hz, Ar**H**, 4H), 5.01 (s, OC**H**₂, 4H), 4.65 (s, OC**H**₂, 4H), 4.59 (d, *J* = 13.9 Hz, ArC**H**ₐₓ, 4H), 4.10 (q, *J* = 7.1 Hz, C**H**₂CH₃, 4H), 3.44 (s, NC**H**₃, 6H), 3.05 (d, *J* = 13.9 Hz, ArC**H**_{eq} , 4H), 1.23 (t, *J* = 7.2 Hz, CH₂C**H**₃, 6H); HRMS (ESI): calculated for C₄H₄₈N₄O₈⁺ [M+H⁺]: 784.3467, measured: 784.3460 [M+H]⁺, 785.3542 [M+2H]⁺ and 807.3366 [M+H+Na]⁺.

### Example 8: Synthesis and characterization of the calix[4]arene of the formula (9):

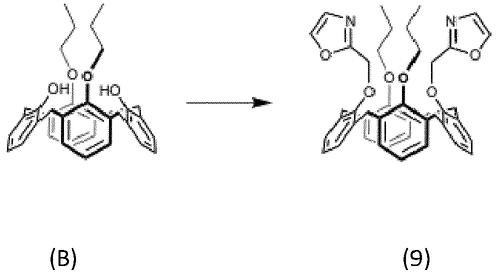

In a 10 mL round bottom flask, the calix[4]arene of the formula (B) (85.6 mg, 0.17 mmol, 1.0 equivalent) was stirred in anhydrous DMF (3 mL) with NaH (60% dispersion in mineral oil, 135 mg, 3.37 mmol, 22 equivalents) for 30 minutes under inert atmosphere. 2-(Chloromethyl)oxazole (89.5 mg, 0.67 mmol, 5.0 equivalents) was then added. The mixture was stirred at reflux for 24 hours under inert atmosphere. After reaction, the mixture was brought to room temperature. The solvent was evaporated under reduced pressure. The residue was dissolved in DCM and then washed with water. The organic layer was evaporated under reduced pressure. The crude product was purified by flash chromatography (DCM/MeOH 95:5 v/v) affording the calix[4]arene of the formula (9) (57 mg, 0.08 mmol) as an oil in 50% yield.

¹H NMR (400 MHz, CDCl₃): δ_{H} (ppm) = 7.65 (s, 2H, Ox**H**), 7.11 (s, 2H, Ox**H**), 6.91 (d, 8 Hz, 4H, Ar**H**), 6.79-6.83 (m, 2H, Ar**H**), 6.31-6.39 (m, 6H, Ar**H**), 5.6 (s, 4H, OC**H**₂Ox), 4.29 (d, *J* = 14 Hz, 4H, ArC**H**₂), 3.67 (t, *J* = 8 Hz, 4H, OC**H**₂Et), 3.14 (d, *J* = 14 Hz, 4H, ArC**H**₂), 1.76-1.85 (m, 4H, CH₂), 0.95 (t, *J* = 8 Hz, 6H, C**H**₃).

### Example 9: Synthesis and characterization of the calix[4]arene of the formula (10):

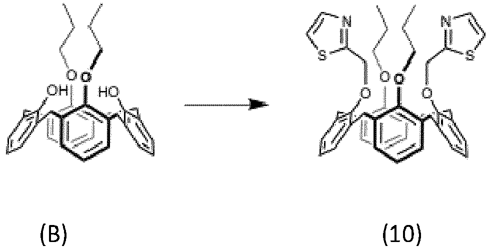

In a 10 mL round bottom flask, the calix[4]arene of the formula (B) (105 mg, 0.21 mmol, 1.0 equivalent) was stirred in anhydrous DMF (3 mL) with NaH (60% dispersion in mineral oil, 165 mg, 4.11 mmol, 23 equiv.) for 30 minutes under inert atmosphere. 2-(Chloromethyl)thiazole (123 mg, 0.82 mmol, 5.6 equivalents) was then added. The mixture was stirred at reflux for 24 hours under inert atmosphere. After reaction, the mixture was brought to room temperature. The solvent was evaporated under reduced pressure. The residue was dissolved in DCM and then washed with water. The organic layer was evaporated under reduced pressure. The crude product was purified by flash chromatography (DCM/MeOH 95:5 v/v) affording the calix[4]arene of the formula (10) (84.0 mg, 0.12 mmol) as an oil in 58% yield.

¹H NMR (400 MHz, CDCl₃): δ_{H} (ppm) = 7.82 (d, *J* = 3.2 Hz, 2H, thioazole**H**), 7.39 (d, *J* = 3.2 Hz, 2H, thioazole**H**), 6.84 (d, *J* = 8 Hz, 4H, Ar**H**), 6.71-6.76 (m, 2H, Ar**H**), 6.47-6.51 (m, 2H, Ar**H**), 6.41 (d, *J* = 8 Hz, 4H, Ar**H**), 5.27 (s, 4H, OC**H**₂thioazole), 4.43 (d, *J* = 14 Hz, 4H, ArC**H**₂), 3.89 (t, *J* = 8 Hz, 4H, OC**H**₂Et), 3.18 (d, *J* = 14 Hz, 4H, ArC**H**₂), 1.77-1.87 (m, 4H, C**H**₂), 0.82 (t, *J* = 8 Hz, 6H, C**H**₃).

1^{ST} Comparative example: The calix[4]arene of the following formula (X) is a comparative example of the present invention:

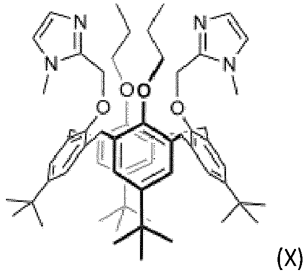

In contrast of the calix[4]arenes of the invention, in the compound of the formula (X) comprises, Y₁, Y₂, Y₃, and Y₄ are tert-butyl groups.

The Ag⁺ complex of the compound of the formula (X) was formed by mixing 600 µL of 0.5 mM solutions of X in CDCl₃ with 600 µL of 0.5 mM AgNO₃ in H₂O for 60 minutes to obtain complex (Xa).

¹H NMR (400 MHz, CDCl₃, 298 K): δ (ppm) = 7.57 (s, Im**H**, 2H), 7.10 (s, Ar**H**, 4H), 6.92 (s, Im**H**, 2H), 6.28 (s, Ar**H**, 4H), 4.94 (s, OCH₂Im, 4H), 3.78 (bd, ArC**H**₂, 4H), 3.68 (bt, OC**H**₂Et, 4H), 2.99 (bd, ArC**H**₂, 4H), 2.85 (s, NC**H**₃, 6H), 1.69-1.75 (m, C**H**₂, 4H), 1.33 (s, ^{t}Bu, 18H), 0.90 (bt, C**H**₃, 6H), 0.76 (s ,^{t}Bu, 18H).

2^{nd} Comparative example: The calix[4]arene of the following formula (XI) is a comparative example of the present invention:

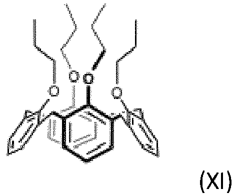

### II - EXPERIMENTS ON cLogP, BINDING OF Cu⁺, TRANSPORT AND DELIVERABILITY, AND CYTOTOXICITY OF CALIX[4]ARENES OF THE PRESENT INVENTION AND THE COMPARATIVE EXAMPLES :

### II - A - Determination of the clogP of the calix[4]arenes of the invention and the comparative examples :

A key requirement for the usability of synthetic transporters (such as the above mentioned ionophores) is their ability to be delivered to the targeted cell. Highly lipophilic molecules which can strongly bind an ion are usually considered good candidates for synthetic transporters. But a high lipophilicity can also prevent the delivery of a compound into a membrane. Thus, a balance has to be achieved between the ability of a molecule to remain in the membrane and carry ions across and getting to this membrane in the first place. For drugs, LogP is the chosen value to assess lipophilicity, which is the logarithm of P, the partition coefficient between water and octanol. A LogP value can be either measured in water/octanol systems or calculated, in the latter case it is referred to as cLogP. Chemical drawing tools such as ChemDraw are able to provide a cLogP value.

In order to assess the lipophilicity of the examples of calix[4]arenes of the present invention and the comparative examples, their cLogP values were determined using the "chemical properties" function in ChemDraw 20.0.

The here below table 1 details these cLogP values for the examples of calix[4]arenes of the invention and the comparative examples (i.e. column "cLogP").

### II - B - Determination of the binding of Cu⁺ :

Binding abilities and complexation constants of the examples of calix[4]arenes of the present invention and the comparative examples with Cu⁺ were evaluated by ¹H NMR titrations at 298 K using copper(I)tetrakis(acetronitrile)tetrafluoroborate.

For example, for each separate calix[4]arene a stock solution of the tested calix[4]arene was prepared in a CD₃CN/CDCl₃, 4/1 mixture. Using this stock solution, a Cu(MeCN)₄BF₄ solution was prepared and added by steps of 0.25 equivalent of copper(I) up until 1 equivalent and then by steps of 0.5 equivalent until a total of at least 2 equivalents. ¹H NMR spectra were recorded at a 400 MHz spectrometer at 298 K for the stock solutions of the calix[4]arenes and after each addition of Cu(MeCN)₄BF₄. Additions were continued until the ¹H NMR signals showed no significant changes anymore.

Calix[4]arenes for which a new set of signals was observed that completely replaced the initial set of signals after the addition of 1 equivalent of Cu(MeCN)₄BF₄ were considered to have an affinity constant (Kₐ) equal or greater than 10⁵ M⁻¹.

Calix[4]arenes for which a gradual change of the chemical shifts of the ¹H NMR signals (> 0.1 ppm for at least one of the signals) was observed upon addition of up to 4-5 equivalents of Cu(MeCN)₄BF₄ were considered to show weak binding.

The here below table 1 details the binding abilities with Cu⁺ for the examples of calix[4]arenes of the invention and the comparative examples (i.e. column "Cu⁺ binding").

More precisely, in this table 1 :
- "strong binding" means an affinity constant (Kₐ) equal or greater than 10⁵ M⁻¹,
- "weak binding" means a change of the chemical shifts of the ¹H NMR signals (> 0.1 ppm for at least one of the signals) is observed upon addition of up to 4-5 equivalents of Cu(MeCN)₄BF₄,
- "not significant" means that no significant changes of chemical shifts (< 0.1 ppm) were observed upon addition of 4-5 equivalents of Cu(MeCN)₄BF₄.

### II - C - 1- Determination of the transport of Cu⁺ by the examples of calix[4]arenes:

The ability of the examples of calix[4]arenes of the invention and the comparative examples to be delivered into a lipidic bilayer membrane of a liposome from a solution and their capacity to then act as a transporter of Cu⁺ was evaluated using bathocuproine disulphonate (hereafter abbreviated "BCS") encapsulated in large unilamellar vesicles (LUVs, also referred to as liposomes) composed of 1-palmitoyl-2-oleoyl-*sn*-glycero-3-phosphocholine and cholesterol in a 7:3 ratio.

BCS is a fluorescent water soluble phenantroline derivative that can be used as fluorescent dye for the sensing of Cu⁺ through the formation of a non-fluorescent 2:1 complex.

Stock solution of lipids were prepared using deacidified CHCl₃ and stored in a freezer. CHCl₃ was deacidified by passing through activated basic alumina. The lipids used to prepare the liposomes were 2-oleoyl-1-palmitoyl-sn-glycero-3-phosphocholine (POPC), (purity ≥ 99%) and cholesterol (purity of 95%). 50 mM sodium phosphate buffer were prepared with Millipore water at pH 7 (± 0.1) by mixing the solution of the mono- and the di-protonated phosphate salts. The solution of the dye (BCS) was prepared at 0.5 mM concentration in phosphate buffer.

The lipids solutions were mixed in different ratios in a 5 mL round bottom flask. The POPC/cholesterol molar ratio was 7:3 in all experiments. The total amount of lipids per batch varied depending on the amount of liposomes solution required for the experiment. After mixing the lipid solutions in a round bottom flask, the chloroform was evaporated under a gentle air flow and the resulting lipid film was dried at high vacuum for at least an hour. 500 µL of the BCS solution in phosphate buffer and a magnetic stir bar were added to the flask. The flask was then sonicated for 30 seconds and placed under magnetic agitation for at least an hour. The solution was frozen with liquid nitrogen and heated back to room temperature with warm water ten times to form large unilamellar vesicles (LUVs). The liposome solution was extruded 29 times, using an extrusion kit equipped with a membrane with 200 nm pores. Subsequently, the solution was eluted with the buffer solution used for the experiment through a size exclusion column (Sephadex G-25) to remove the external BCS and then diluted with phosphate buffer to obtain a concentration of 0.4 mM in lipids.

Solutions of the calix[4]arenes were prepared at a concentration of 0.24 mM in DMSO and 5 µL of the solution was added 2 minutes before each transport measurement. This results in a ratio of calix[4]arene to lipid of 1:1000, corresponding to a 0.4 µM concentration of the tested calix[4]arenes.

Alternatively, calix[4]arenes were pre-incorporated by combining the calix[4]arene (dissolved in chloroform) with the lipids prior to the evaporation of the chloroform to obtain a lipid film. The concentrations and volumes were chosen to obtain a calix[4]arene to lipid of 1:1000.

Fluorescence measurements were performed with a Horiba FluoroMax 4 spectrometer using a 4-faced quartz cuvette (10 mm × 10 mm). For the Cu⁺ transport assay, the excitation was set at 278 nm and emission was recorded at 393 nm. These values correspond to the excitation and emission maxima of BCS encapsulated inside LUVs.

The Cu⁺ solutions added to the liposomes were obtained by dissolving Cu(MeCN)₄BF₄ in MeCN at 15.25 mM concentration. The copper solution was then diluted by a factor 5 in a phosphate buffer during the first 30 seconds of the experiment to reach a copper concentration of 3.05 mM. 50 µL of the diluted copper solution was added to 3 mL of liposomes, resulting in a copper gradient of 50 µM. The fluorescence level was monitored for 600 seconds after the addition of the copper solution.

For each run the initial plateau and the initial vertical drop (due to the binding of the exterior dye with Cu⁺ and the dilution, 1-4 seconds after the addition) were removed. Then the signal was normalised by dividing the fluorescence values (*F*) by the value at t = 0 second (*F₀*)*.* The average of 3 normalised runs (*F*/*F₀*) was then calculated and plotted over time and final *F*/*F₀* levels (after 600 s) were determined.

The here below table 1 details the abilities of Cu⁺ transport for the examples of calix[4]arenes of the invention and the comparative examples (i.e. column "Cu⁺ transport").

More precisely, in this table 1 :
- "Yes" means that a significant decrease of the fluorescence of encapsulated BCS was observed upon addition of Cu⁺ to the liposomes (*F*/*F₀* < 0.65 after 600 s),
- "No" means that no significant decrease of the fluorescence of encapsulated BCS was observed upon addition of Cu⁺ to the liposomes (*F*/*F₀* > 0.65 after 600 s) when the calix[4]arene was either delivered from a solution in DMSO to liposomes or pre-incorporated into the liposomes.

### II - C - 2- Determination of the transport of Ag⁺ by the calix[4]arene of the formula (2):

Liposomes with BCS encapsulated were prepared as described above in the part entitled "II - C - 1- Determination of the transport of Cu⁺ by the examples of calix[4]arenes" and measurements were performed as described in this part.

A Ag⁺ solution was prepared by dissolving AgNO₃ in a phosphate buffer at 3.05 mM concentration. After the first 30 seconds of the experiment, 50 µL of the Ag⁺ solution was added to 3 mL of liposomes, resulting in a silver gradient of 50 µM. The fluorescence level was monitored for 600 seconds after the addition of the copper solution.

When a solution of DMSO with the calix[4]arene of the formula (2) was added to the liposomes (5 µL, to obtain a 1:1000 transport to lipid ratio) the decrease in fluorescence intensity of BCS was much faster (F/F₀ = 0.55 after 100 s) compared to when DMSO without calix[4]arene of the formula (2) (5 µL) was added (F/F₀ = 0.85 after 100 s).

### II - D - Determination of the deliverability:

The ability of the examples of calix[4]arenes of the present invention and the comparative examples to be inserted into the membrane of liposomes after their formation was tested.

For that, the procedure in which calix[4]arenes were added to the liposomes from a solution in DMSO, as detailed in part "II - C - 1 -Determination of the transport of Cu⁺ by the examples of calix[4]arenes", was followed and transport curves were recorded.

Calix[4]arenes showing poor or no activity via this procedure were tested via pre-incorporation.

More precisely, the calix[4]arene (dissolved in chloroform) was combined with the lipids prior to the evaporation of the chloroform to obtain a lipid film. The concentrations and volumes were chosen to obtain a calix[4]arene to lipid of 1:1000.

The here below table 1 details the deliverability of the tested calix[4]arenes and the comparative examples to liposomes from a solution in DMSO (i.e. column « deliverability »).

More precisely, in this table 1 :
- "Yes" means that a significant decrease of the fluorescence of encapsulated BCS was observed upon addition of Cu⁺ to the liposomes when the calix[4]arene was added to the liposomes from a solution in DMSO (*F*/*F₀* < 0.65 after 600 s),
- "Medium" means that the final level reached was much lower upon pre-incorporation (*F*/*F₀* < 0.4 after 600 s) than when adding the calix[4]arene to the liposomes from a solution in DMSO (*F*/*F₀* = 0.4-0.65 after 600 s),
- "No" means that no significant decrease of the fluorescence of encapsulated BCS was observed (*F*/*F₀* > 0.65 after 600 s) when adding the calix[4]arene to the liposomes from a solution in DMSO, but that upon pre-incorporation good transport was observed (*F*/*F₀* < 0.4 after 600 s),
- "-" means that no transport was observed either by pre-incorporation or when adding the calix[4]arene to the liposomes from a solution in DMSO, indicating that the calix[4]arene is not active as Cu⁺ transport in the described method and that this method can thus not be used to verify the deliverability of the calix[4]arene.

**Table 1 detailing the cLogP values and abilities of binding or transporting of some calix[4]arenes of the invention and the comparative examples**

| **Formula of the calix[4]arene / example** | **cLogP** | **Cu⁺ binding** | **Cu⁺ transport** | **Deliverability** |
|---|---|---|---|---|
| (2) / example 1 | 9.2 | Strong binding | Yes | Yes |
| (5) / example 2 | 7.1 | Strong binding | Yes | Yes |
| (3) / example 3 | 8.7 | Strong binding | Yes | Yes |
| (6) / example 4 | 5.9 | Strong binding | Yes | Yes |
| (7) / example 5 | 8.7 | Strong binding | Yes | Yes |
| (8) / example 6 | 12.4 | Strong binding | Yes | Yes |
| (4) / example 7 | 7.4 | Strong binding | Yes | Yes |
| (9) / example 8 | 9.4 | Weak binding | Yes | Medium |
| (10) / example 9 | 10.4 | Weak binding | Yes | Medium |
| (X) / 1^{st} comparative example | 16.5 | Strong binding | Yes | No |
| (XI) / 2^{nd} comparative example | 12.7 | Not significant | No | - |

Furthermore, as alternative to the addition of the calix[4]arene of the formula (2) and the 1^{st} comparative example of the formula (X) from a solution in DMSO, the delivery from liposomes was tested.

For the delivery studies with liposomes, two batches of liposomes were prepared. Liposomes with BCS encapsulated were prepared as above described and liposomes without any BCS but with either the calix[4]arene of the formula (2) or the 1^{st} comparative example of the formula (X) pre-incorporated at a 1:500 transporter to lipid ratio were prepared as described above. 1.5 mL of each batch of liposomes were added to a cuvette and left to stir for 5 minutes in the spectrometer before starting the experiments, using the settings as described above.

When liposomes with BCS were mixed with liposomes containing the calix[4]arene of the formula (2), a good transport activity was observed, indicating that the calix[4]arene of the formula (2) can be delivered to liposomal membranes from other liposomes. In contrast, when liposomes with BCS were mixed with liposomes containing the 1^{st} comparative example of the formula (X), no transport activity was observed, indicating that this lipophilic comparative example of the formula (X) cannot be delivered from liposomes either.

In view of the table 1, the calix[4]arenes of the formulas (2), (3) and (4) exhibit good abilities for binding and transport of Cu⁺ and to be delivered from a DMSO solution. Moreover, their lipophilicities are not too high (i.e. between 7.4 and 9.2). These calix[4]arenes are preferred calix[4]arenes of the invention.

In contrast, although the comparative example of formula (X) binds Cu⁺ strongly and is able to transport, it is not an interesting calix[4]arene for anticancer therapy because of its high lipophilicity (cLogP =16.5) and the absence of deliverability. Indeed, the comparative example of the formula (X) is neither deliverable from a solution of DMSO nor from liposomes.

### III - EXPERIMENTS OF CELL PROLIFERATION:

### III - A - Inhibition of A549 cell proliferation:

The inhibition of a A549 human lung cancer cell line proliferation by the calix[4]arene of the formula (2) was determined by exposure of said A549 human lung cancer cell line to a concentration of 2 µM of the calix[4]arene of the formula (2) until 72 hours. The absolute number of cells were counted in the treated and untreated conditions before treatment (N₀) and after 72 hours (Nₜ).

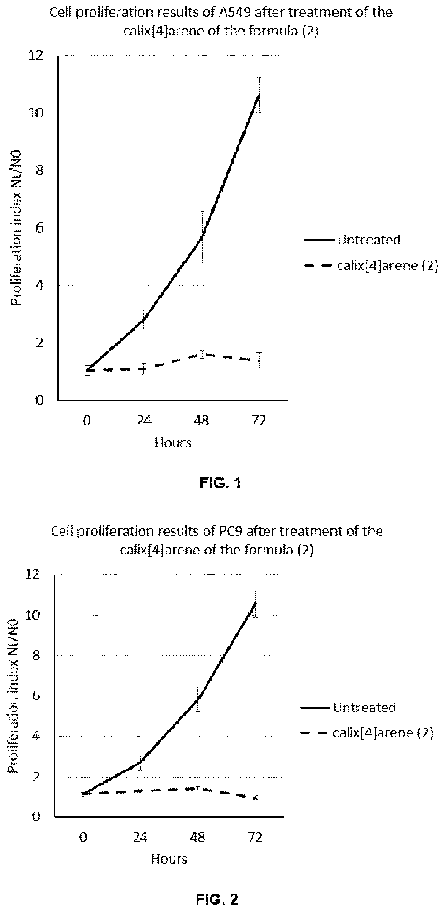

The figure 1 is a graph showing :
- the profile of the proliferation index Nₜ/N₀ of the A549 cells after exposure with the calix[4]arene of the formula (2) with respect to the time (i.e. the curve entitled "calix[4]arene (2)"),
- the profile of the proliferation index Nₜ/N₀ of the A549 cells without any exposure with the calix[4]arene of the formula (2) with respect to the time (i.e. the curve entitled "untreated").

"Nₜ" is number of cells at time "t" and "N₀" is number of cells before treatment.

In view of the curves of the graph of the figure 1, the calix[4]arene of the formula (2) clearly inhibits the proliferation of the A549 human lung cancer cells.

### III - B - Inhibition of PC9 cell proliferation:

The inhibition of a PC9 human lung cancer cell line proliferation by the calix[4]arene of the formulation (2) was determined by exposure of said PC9 human lung cancer cell line to a concentration of 3 µM of the compound of the formula (2) until 72 hours. The absolute number of cells were counted in the treated and untreated conditions before treatment (N₀) and after 72 hours (Nₜ).

The figure 2 is a graph showing :
- the profile of the proliferation index Nₜ/N₀ of the PC9 cells after exposure with the calix[4]arene of the formula (2) with respect to the time (i.e. the curve entitled "calix[4]arene (2)"),
- the profile of the proliferation index Nₜ/N₀ of the PC9 cells without any exposure with the calix[4]arene of the formula (2) with respect to the time (i.e. the curve entitled "untreated").

In view of the curves of the graph of the figure 2, the calix[4]arene of the formula (2) clearly inhibits the proliferation of the PC9 human lung cancer cells.

### III - C - Inhibition of H322 cell proliferation:

The inhibition of a H322 lung carcinoma cell line proliferation by the calix[4]arene of the formula (2) was determined by exposure of said H322 lung carcinoma cell line to a concentration of 3 µM of the calix[4]arene of the formula (2) until 72 hours. The absolute number of cells were counted in the treated and untreated conditions before treatment (N₀) and after 72 hours (Nₜ).

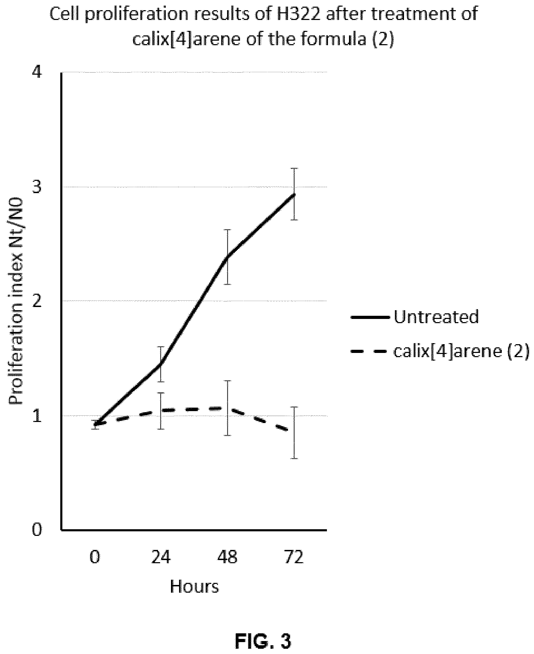

The figure 3 is a graph showing :
- the profile of the proliferation index Nₜ/N₀ of the H322 cells after exposure with the calix[4]arene of the formula (2) with respect to the time (i.e. the curve entitled "calix[4]arene (2)"),
- the profile of the proliferation index Nₜ/N₀ of the H322 cells without any exposure with the calix[4]arene of the formula (2) with respect to the time (i.e. the curve entitled "untreated").

In view of the curves of the graph of the figure 3, the calix[4]arene of the formula (2) clearly inhibits the proliferation of the H322 lung carcinoma cells.

### IV - EXPERIMENTS OF CYTOXICITY ON THE CALIX[4]ARENE OF THE FORMULA (2), DETERMINATION OF IC₅₀:

### IV - A- Experiments on malignant H322, A549 and PC9 cells

H322 lung carcinoma cells were seeded in 96-well plates together with the calix[4]arene of the formula (2). Cells were grown in RPMI-1640 supplemented with GlutaMAX^{™} (Gibco^{™}) and fetal calf serum (FCS, 10%) and treated with a broad range of concentrations of the calix[4]arene of the formula (2). After 72 hours of treatment, the cytotoxicity was measured by means of the IC₅₀ value, using the kit "CellTiter 96R AQᵤₒᵤₛ One Solution Cell Proliferation Assay (MTS)" from Promega^{™}. Following 30 minutes of incubation with the MTS reagent, the endpoint measurements were done in triplicate at 485nm, using a fluorescence plate reader.

A549 human lung cancer cells were seeded in 96-well plates together with the calix[4]arene of the formula (2). Cells were grown in RPMI-1640 supplemented with GlutaMAX^{™} (Gibco^{™}) and fetal calf serum (FCS, 10%) and treated with a broad range of concentrations of the calix[4]arene of the formula (2). After 72 hours of treatment, the cytotoxicity was measured by means of the IC₅₀ value, using the kit "CellTiter 96R AQᵤₒᵤₛ One Solution Cell Proliferation Assay (MTS)" from Promega^{™}. Following 30 minutes of incubation with the MTS reagent, the endpoint measurements were done in triplicate at 485nm, using a fluorescence plate reader.

PC9 human lung cancer cells were seeded in 96-well plates together with the calix[4]arene of the formula (2). Cells were grown in RPMI-1640 supplemented with GlutaMAX^{™} (Gibco^{™}) and fetal calf serum (FCS, 10%) and treated with a broad range of concentrations of the calix[4]arene of the formula (2). After 72 hours of treatment, the cytotoxicity was measured by means of the IC₅₀ value, using the kit "CellTiter 96R AQᵤₒᵤₛ One Solution Cell Proliferation Assay (MTS)" from Promega^{™}. Following 30 minutes of incubation with the MTS reagent, the endpoint measurements were done in triplicate at 485nm, using a fluorescence plate reader.

The here below table 2 details the mean IC₅₀s using MTS cytotoxicity assay on H322, A549 and PC9 cells for the calix[4]arene of the formula (2) and the confidence interval (statistical significance 95%) of the IC₅₀ obtained with GraphPad Prism software.

**Table 2 of the IC₅₀s using MTS assay on H322, A549 and PC9 cells for the calix[4]arene of the formula (2)**

| **Cell lines** | **Mean IC₅₀ (µM)** | **Confidence Interval (95%) for IC₅₀ (µM) values** |
|---|---|---|
| **H322** | 1.84 | 1.69 to 2.042 |
| **A549** | 1.56 | 1.45 to 1.69 |
| **PC9** | 2.06 | 1.79 to 2.38 |

In view of the table 2, the calix[4]arene of the formula (2) exhibits a high antitumor activity on H322, A549 and PC9 cells in the micromolar range, which are values in the same range as for other anticancer chemotherapeutic drugs.

### IV - B- Experiments on non-malignant MRCS-SVII, MSC and HaCaT cells

Non-malignant MRC5-SVII cells were seeded in 96-well plates together with the calix[4]arene of the formula (2). Cells were grown in Minimal Essential Medium - Non Essential Amino Acids (MEM NEAA) culture medium from Gibco^{™}, supplemented with sodium pyruvate (1µM), and fetal calf serum (FCS, 10%) and treated with a broad range of concentrations of the calix[4]arene of the formula (2). After 72 hours of treatment, the cytotoxicity was measured by means of the IC₅₀ value, using the kit "CellTiter 96R AQᵤₒᵤₛ One Solution Cell Proliferation Assay (MTS)" from Promega^{™}. Following 30 minutes of incubation with the MTS reagent, the endpoint measurements were done in triplicate at 485 nm, using a fluorescence plate reader.

Non-malignant MSC cells were seeded in 96-well plates together with the calix[4]arene of the formula (2). Cells were grown in Mesenchymal Stem Cell Growth Medium (C-28009 from Promega^{™}), and treated with a broad range of concentrations of the calix[4]arene of the formula (2). After 72 hours of treatment, the cytotoxicity was measured by means of the IC₅₀ value, using the kit "CellTiter 96R AQᵤₒᵤₛ One Solution Cell Proliferation Assay (MTS)" from Promega^{™}. Following 30 minutes of incubation with the MTS reagent, the endpoint measurements were done in triplicate at 485 nm, using a fluorescence plate reader.

Non-malignant HaCaT cells were seeded in 96-well plates together with the calix[4]arene of the formula (2). Cells were grown in RPMI-1640 supplemented with GlutaMAX^{™} (Gibco^{™}) and fetal calf serum (FCS, 20%) and treated with a broad range of concentrations of the calix[4]arene of the formula (2). After 72 hours of treatment, the cytotoxicity was measured by means of the IC₅₀ value, using the kit "CellTiter 96R AQᵤₒᵤₛ One Solution Cell Proliferation Assay (MTS)" from Promega^{™}. Following 30 minutes of incubation with the MTS reagent, the endpoint measurements were done in triplicate at 485 nm, using a fluorescence plate reader.

The here below table 3 details the IC₅₀s using MTS cytotoxicity assay on MRCS-SVII, MSC and HaCaT cells for the calix[4]arene of the formula (2) and the confidence interval (statistical significance 95%) of the IC₅₀ obtained with GraphPad Prism software.

**Table 3 of the IC₅₀s using MTS assay on MRC5-SVII, MSC and HaCat cells for the calix[4]arene of the formula (2)**

| **Cell lines** | **Mean IC₅₀ (µM)** | **Confidence Interval (95%) for IC₅₀ (µM) values** |
|---|---|---|
| **MRCS-SVII** | 1.03 | 0.99 to 1.06 |
| **MSC** | 1.01 | 0.93 to 1.09 |
| **HaCaT** | 1.05 | 0.93 to 1.18 |

In view of the table 3, the calix[4]arene of the formula (2) exhibits a toxicity on non-malignant MRC5-SVII, MSC and HaCat cells, in the same range as for cancer cells (see Table 2).

### IV - C- Experiments on 60 malignant cell lines

In these experiments, the IC₅₀s values were determined for the calix[4]arene of the formula (2) and for a reference compound which was bortezomib (i.e. an anticancer compound which inhibits proteasome).

The cytotoxicity was measured by means of the IC₅₀ value, using the PROLiFiler-Cell Panel Screening (Reaction Biology^{™}).

Cells were cultured in different media. The here below table 4 details the corresponding medium for each tested malignant cell.

For the assays, cells were seeded in white cell culture-treated flat and clear bottom 384-well multiwall plates and incubated at 37°C overnight before the calix[4]arene of the formula (2) or the reference compound were added. After incubation for 72 hours at 37°C at 5% or 10% CO₂ depending on the medium, cell plates were equilibrated to room temperature (i.e. 20°C) for one hour, CellTiterGlo^{®} reagent from the firm Promega was added and luminescence was measured approximately an hour later using a luminometer.

Raw data were converted into percent cell viability relative to the high and low control, which were set to 100% and 0%, respectively. IC₅₀ calculation was performed using GraphPad Prism software with a variable slope sigmoidal response fitting model using 0% viability as bottom constraint and 100% viability as top constraint.

The here below table 4 details the medium of culture for each tested malignant cell line.

**Table 4 detailing the medium of culture of the calix[4]arene of the formula (2) and the reference compound for each malignant cell line**

| **No.** | **Entity** | **Cell line** | **medium** |
|---|---|---|---|
| **1** | Brain | A172 | DMEM + 10% FCS |
| **2** | Skin | A2058 | DMEM + 10% FCS |
| **3** | Skin | A375 | DMEM + 10% FCS |
| **4** | Kidney | A498 | DMEM + 10% FCS |
| **5** | Lung | A549 | DMEM + 10% FCS |
| **6** | Pancreas | AsPC-1 | DMEM + 10% FCS |
| **7** | Lung | BEN | DMEM + 10% FCS |
| **8** | Breast | BT-20 | RPMI-1640 + 10% FCS |
| **9** | Kidney | Caki-1 | DMEM + 10% FCS |
| **10** | Kidney | Caki-2 | DMEM + 10% FCS |
| **11** | Colon | Colo 205 | DMEM + 10% FCS |
| **12** | Lung | COR-L279 | RPMI-1640 + 10% FCS |
| **13** | Ovary | COV434 | DMEM + 10% FCS |
| **14** | Colon | DLD-1 | DMEM + 10% FCS |
| **15** | Prostate | DU-145 | DMEM + 10% FCS |
| **16** | Lung | DV90 | RPMI-1640 + 10% FCS |
| **17** | Breast | EFM-192A | RPMI-1640 + 10% FCS |
| **18** | Ovary | EFO-27 | RPMI-1640 + 10% FCS |
| **19** | Lung | EPLC-272H | RPMI-1640 + 10% FCS |
| **20** | Lung | H1299 | DMEM + 10% FCS |
| **21** | Brain | H4 | DMEM + 10% FCS |
| **22** | Lung | H460 | DMEM + 10% FCS |
| **23** | Breast | HCC 1569 | RPMI-1640 + 10% FCS |
| **24** | Breast | HCC38 | RPMI-1640 + 10% FCS |
| **25** | Lung | HCC827 | RPMI-1640 + 10% FCS |
| **26** | Colon | HCT116 | DMEM + 10% FCS |
| **27** | Colon | HCT-15 | RPMI-1640 + 10% FCS |
| **28** | Ovary | HeLa | DMEM + 10% FCS |
| **29** | Liver | Hep3B2.1-7 | DMEM + 10% FCS |
| **30** | Blood | HL-60 | RPMI-1640 + 10% FCS |
| **31** | Fibrosarcoma | HT-1080 | DMEM + 10% FCS |
| **32** | Colon | HT-29 | DMEM + 10% FCS |
| **33** | Liver | HuH7 | DMEM + 10% FCS |
| **34** | Breast | JIMT-1 | DMEM + 10% FCS |
| **35** | Blood | K562 | RPMI-1640 + 10% FCS |
| **36** | Blood | KARPAS 299 | RPMI-1640 + 10% FCS |
| **37** | Prostate | LnCap | RPMI-1640 + 10% FCS |
| **38** | Lung | LOU-NH91 | RPMI-1640 + 10% FCS |
| **39** | Skin | MDA MB 435 | RPMI-1640 + 10% FCS |
| **40** | Breast | MDA-MB-468 | DMEM + 10% FCS |
| **41** | Stomach | MKN-1 | RPMI-1640 + 10% FCS |
| **42** | Blood | MOLM-13 | RPMI-1640 + 10% FCS |
| **43** | Blood | MV4-11 | RPMI-1640 + 10% FCS |
| **44** | Lung | NCI-H1563 | RPMI-1640 + 10% FCS |
| **45** | Lung | NCI-H1573 | RPMI-1640 + 10% FCS |
| **46** | Lung | NCI-H1838 | RPMI-1640 + 10% FCS |
| **47** | Ovary | SiHa | DMEM + 10% FCS |
| **48** | Ovary | SK-OV3 | DMEM + 10% FCS |
| **49** | Stomach | SNU-1 | RPMI-1640 + 10% FCS |
| **50** | Ovary | SNU840 | RPMI-1640 + 10% FCS |
| **51** | Brain | SW-1783 | DMEM + 10% FCS |
| **52** | Colon | SW480 | DMEM + 10% FCS |
| **53** | Colon | SW620 | DMEM + 10% FCS |
| **54** | Colon | SW948 | DMEM + 10% FCS |
| **55** | Colon | T84 | DMEM + 10% FCS |
| **56** | Brain | T98G | DMEM + 10% FCS |
| **57** | Brain | U118MG | DMEM + 10% FCS |
| **58** | Brain | U251MG | DMEM + 10% FCS |
| **59** | Bone | U2OS | DMEM + 10% FCS |
| **60** | Brain | U87MG | DMEM + 10% FCS |

In this table 4:
- "DMEM" is the abbreviation for Dulbecco's Modified Eagle's Medium,
- "FCS" is the abbreviation for fetal calf serum,
- "RPMI-1640" is the abbreviation for Roswell Park Memorial Institute.

The here below table 5 details for each tested malignant cell line the IC₅₀ values for the calix[4]arene of the formula (2) and the reference compound bortezomib.

**Table 5 detailing the IC50 values for the calix[4]arene of the formula (2) and the reference compound for each tested malignant cell line**

| **No.** | **Entity** | **Cell line** | **IC50 [M] calix[4]arene (2)** | **IC50 [M] bortezomib** |
|---|---|---|---|---|
| **1** | Brain | A172 | 1.21E-06 | 4.77E-09 |
| **2** | Skin | A2058 | 9.38E-07 | 9.24E-09 |
| **3** | Skin | A375 | 1.17E-06 | 5.44E-09 |
| **4** | Kidney | A498 | 1.72E-06 | 4.53E-09 |
| **5** | Lung | A549 | 1.04E-06 | 1.37E-08 |
| **6** | Pancreas | AsPC-1 | 8.34E-07 | 1.25E-08 |
| **7** | Lung | BEN | 3.68E-06 | 5.31E-08 |
| **8** | Breast | BT-20 | 1.27E-06 | 2.95E-08 |
| **9** | Kidney | Caki-1 | 3.24E-06 | 5.37E-09 |
| **10** | Kidney | Caki-2 | 2.61E-06 | 1.28E-08 |
| **11** | Colon | Colo 205 | 1.81E-06 | 6.79E-09 |
| **12** | Lung | COR-L279 | 1.51E-06 | 4.94E-09 |
| **13** | Ovary | COV434 | 7.32E-07 | 9.24E-09 |
| **14** | Colon | DLD-1 | 1.08E-06 | 6.83E-09 |
| **15** | Prostate | DU-145 | 1.93E-06 | 6.80E-09 |
| **16** | Lung | DV90 | 2.16E-06 | 7.37E-09 |
| **17** | Breast | EFM-192A | 5.45E-06 | 4.75E-09 |
| **18** | Ovary | EFO-27 | 1.52E-06 | 1.32E-08 |
| **19** | Lung | EPLC-272H | 1.47E-06 | 7.17E-09 |
| **20** | Lung | H1299 | 1.30E-06 | 8.42E-09 |
| **21** | Brain | H4 | 2.90E-06 | 8.36E-09 |
| **22** | Lung | H460 | 7.27E-07 | 2.88E-08 |
| **23** | Breast | HCC 1569 | 2.96E-06 | 5.15E-09 |
| **24** | Breast | HCC38 | 2.53E-06 | 5.41E-09 |
| **25** | Lung | HCC827 | 1.72E-06 | 8.40E-09 |
| **26** | Colon | HCT116 | 9.50E-07 | 6.78E-09 |
| **27** | Colon | HCT-15 | 2.02E-06 | 9.25E-09 |
| **28** | Ovary | HeLa | 1.70E-06 | 1.87E-08 |
| **29** | Liver | Hep3B2.1-7 | 7.81E-07 | 7.98E-09 |
| **30** | Blood | HL-60 | 1.45E-06 | 3.73E-09 |
| **31** | Fibrosarcoma | HT-1080 | 1.90E-06 | 1.38E-08 |
| **32** | Colon | HT-29 | 1.20E-06 | 5.99E-09 |
| **33** | Liver | HuH7 | 5.43E-07 | 4.30E-09 |
| **34** | Breast | JIMT-1 | 2.70E-06 | 8.57E-09 |
| **35** | Blood | K562 | 1.89E-06 | 1.46E-08 |
| **36** | Blood | KARPAS 299 | 1.48E-06 | 3.96E-09 |
| **37** | Prostate | LnCap | 1.19E-06 | 1.41E-08 |
| **38** | Lung | LOU-NH91 | 6.52E-07 | 7.04E-09 |
| **39** | Skin | MDA MB 435 | 2.77E-06 | 4.30E-09 |
| **40** | Breast | MDA-MB-468 | 7,41E-07 | 4.20E-09 |
| **41** | Stomach | MKN-1 | 2,16E-06 | 4.32E-09 |
| **42** | Blood | MOLM-13 | 1,71E-06 | 3.91E-09 |
| **43** | Blood | MV4-11 | 6,71E-07 | 3.13E-09 |
| **44** | Lung | NCI-H1563 | 3,44E-06 | 1.95E-08 |
| **45** | Lung | NCI-H1573 | 2,21E-06 | 1.67E-08 |
| **46** | Lung | NCI-H1838 | 3,48E-06 | 3.41E-08 |
| **47** | Ovary | SiHa | 1,25E-06 | 7.41E-09 |
| **48** | Ovary | SK-OV3 | 1,62E-06 | 2.40E-08 |
| **49** | Stomach | SNU-1 | 2,55E-06 | 2.88E-08 |
| **50** | Ovary | SNU840 | 1,15E-06 | 5.89E-09 |
| **51** | Brain | SW-1783 | 3,38E-06 | 8.60E-09 |
| **52** | Colon | SW480 | 1,52E-06 | 5.29E-09 |
| **53** | Colon | SW620 | 1,76E-06 | 4.74E-09 |
| **54** | Colon | SW948 | 2,15E-06 | 8.88E-09 |
| **55** | Colon | T84 | 2,44E-06 | 1.21E-08 |
| **56** | Brain | T98G | 3,11E-06 | 1.14E-08 |
| **57** | Brain | U118MG | 2,03E-06 | 4.14E-09 |
| **58** | Brain | U251MG | 3,53E-06 | 9.16E-09 |
| **59** | Bone | U2OS | 1,06E-06 | 5.83E-09 |
| **60** | Brain | U87MG | 2,46E-06 | 8.02E-09 |

In view of the table 5, the calix[4]arene of the formula (2) exhibits for all the tested malignant cells an antitumor activity, in the same micromolar range as for the lung cancer cell lines (see table 2).

### V - EXPERIMENTS OF CYTOXICITY ON THE 2^{ND} COMPARATIVE EXAMPLE OF THE FORMULA (XI), DETERMINATION OF IC₅₀:

### V - A- Experiments on malignant H322, A549 and PC9 cells

H322 lung carcinoma cells were seeded in 96-well plates together with the compound of the 2^{nd} comparative example of the formula (XI). Cells were grown in RPMI-1640 supplemented with GlutaMAX^{™} (Gibco^{™}) and fetal calf serum (FCS, 10%) and treated with a broad range of concentrations of the 2^{nd} comparative example of the formula (XI). After 72 hours of treatment, the cytotoxicity was measured by means of the IC₅₀ value, using the kit "CellTiter 96R AQᵤₒᵤₛ One Solution Cell Proliferation Assay (MTS)" from Promega^{™}. Following 30 minutes of incubation with the MTS reagent, the endpoint measurements were done in triplicate at 485 nm, using a fluorescence plate reader.

A549 human lung cancer cells were seeded in 96-well plates together with the compound of the 2^{nd} comparative example of the formula (XI). Cells were grown in RPMI-1640 supplemented with GlutaMAX^{™} (Gibco^{™}) and fetal calf serum (FCS, 10%) and treated with a broad range of concentrations of the 2^{nd} comparative example of the formula (XI). After 72 hours of treatment, the cytotoxicity was measured by means of the IC₅₀ value, using the kit "CellTiter 96R AQᵤₒᵤₛ One Solution Cell Proliferation Assay (MTS)" from Promega^{™}. Following 30 minutes of incubation with the MTS reagent, the endpoint measurements were done in triplicate at 485 nm, using a fluorescence plate reader.

PC9 human lung cancer cells were seeded in 96-well plates together with the 2^{nd} comparative example of the formula (XI). Cells were grown in RPMI-1640 supplemented with GlutaMAX^{™} (Gibco^{™}) and fetal calf serum (FCS, 10%) and treated with a broad range of concentrations of the compound of the 2^{nd} comparative example of the formula (XI). After 72 hours of treatment, the cytotoxicity was measured by means of the IC₅₀ value, using the kit "CellTiter 96R AQᵤₒᵤₛ One Solution Cell Proliferation Assay (MTS)" from Promega^{™}. Following 30 minutes of incubation with the MTS reagent, the endpoint measurements were done in triplicate at 485 nm, using a fluorescence plate reader.

The here below table 6 details the IC₅₀s using MTS cytotoxicity assay on H322, A549 and PC9 cells for the 2^{nd} comparative example of the formula (XI).

**Table 6 of the IC₅₀s using MTS assay on H322, A549 and PC9 cells for the 2^{nd} comparative example of the formula (XI)**

| **Cell lines** | **IC₅₀ (µM)** |
|---|---|
| **H322** | > 50 µM |
| **A549** | > 50 µM |
| **PC9** | > 50 µM |

The 2^{nd} comparative example of the formula (XI) shows no signs of significant cytotoxicity on the lung cancer cell lines.

### V - B- Experiments on non-malignant MRCS-SVII cells

Non-malignant MRC5-SVII cells were seeded in 96-well plates together with the 2^{nd} comparative example of the formula (XI). Cells were grown in Minimal Essential Medium - Non Essential Amino Acids (MEM NEAA) culture medium from Gibco^{™}, supplemented with sodium pyruvate (1µM), and fetal calf serum (FCS, 10%) and treated with a broad range of concentrations of the 2^{nd} comparative example of the formula (XI). After 72 hours of treatment, the cytotoxicity was measured by means of the IC₅₀ value, using the kit "CellTiter 96R AQᵤₒᵤₛ One Solution Cell Proliferation Assay (MTS)" from Promega^{™}. Following 30 minutes of incubation with the MTS reagent, the endpoint measurements were done in triplicate at 485 nm, using a fluorescence plate reader.

The here below table 7 details the IC₅₀ using MTS cytotoxicity assay on MRC5-SVII cells for the 2^{nd} comparative example of the formula (XI):

**Table 7 of the IC₅₀s using MTS assay on MRC5-SVII cells for the 2nd comparative example of the formula (XI)**

| **Cell lines** | **IC₅₀ (µM)** |
|---|---|
| **MRCS-SVII** | > 20 µM |

The 2^{nd} comparative example of the formula (XI) shows no signs of significant cytotoxicity on MRC5-SVII cell lines.

### VI - EXPERIMENTS OF CYTOXICITY ON THE 1^{ST} COMPARATIVE EXAMPLE OF THE FORMULA (X) BASED ON IC₅₀:

### Experiments on malignant H322, A549 and PC9 cells

H322 lung carcinoma cells were seeded in 96-well plates together with the 1^{st} comparative example of the formula (X). Cells were grown in RPMI-1640 supplemented with GlutaMAX^{™} (Gibco^{™}) and fetal calf serum (FCS, 10%) and treated with a broad range of concentrations of the compound of the formula (X). After 72 hours of treatment, the cytotoxicity was measured by means of the IC₅₀ value, using the kit "CellTiter 96R AQᵤₒᵤₛ One Solution Cell Proliferation Assay (MTS)" from Promega^{™}. Following 30 minutes of incubation with the MTS reagent, the endpoint measurements were done in triplicate at 485 nm, using a fluorescence plate reader.

A549 human lung cancer cells were seeded in 96-well plates together with 1^{st} comparative example of the formula (X). Cells were grown in RPMI-1640 supplemented with GlutaMAX^{™} (Gibco^{™}) and fetal calf serum (FCS, 10%) and treated with a broad range of concentrations of the 1^{st} comparative example of the formula (X). After 72 hours of treatment, the cytotoxicity was measured by means of the IC₅₀ value, using the kit "CellTiter 96R AQᵤₒᵤₛ One Solution Cell Proliferation Assay (MTS)" from Promega^{™}. Following 30 minutes of incubation with the MTS reagent, the endpoint measurements were done in triplicate at 485 nm, using a fluorescence plate reader.

PC9 human lung cancer cells were seeded in 96-well plates together with the 1^{st} comparative example of the formula (X). Cells were grown in RPMI-1640 supplemented with GlutaMAX^{™} (Gibco^{™}) and fetal calf serum (FCS, 10%) and treated with a broad range of concentrations of the 1^{st} comparative example of the formula (X). After 72 hours of treatment, the cytotoxicity was measured by means of the IC₅₀ value, using the kit "CellTiter 96R AQᵤₒᵤₛ One Solution Cell Proliferation Assay (MTS)" from Promega^{™}. Following 30 minutes of incubation with the MTS reagent, the endpoint measurements were done in triplicate at 485 nm, using a fluorescence plate reader.

The here below table 8 details the IC₅₀s using MTS cytotoxicity assay on H322, A549 and PC9 cells for the 1^{st} comparative example of the formula (X):

**Table 8 of the IC₅₀s using MTS assay on H322, A549 and PC9 cells for the 1st comparative example of the formula (X)**

| **Cell lines** | **IC₅₀ (µM)** |
|---|---|
| **H322** | > 50 µM |
| **A549** | > 50 µM |
| **PC9** | > 50 µM |

The 1^{st} comparative example of the formula (X) shows no signs of significant cytotoxicity on the lung cancer cell lines.

### VII - EXPERIMENTS OF CYTOXICITY ON OTHER CALIX[4]ARENES OF THE INVENTION BASED ON IC₅₀:

### Experiments on malignant H322 cells

H322 lung carcinoma cells were seeded in 96-well plates together with the tested calix[4]arenes. Cells were grown in RPMI-1640 supplemented with GlutaMAX^{™} (Gibco^{™}) and fetal calf serum (FCS, 10%) and treated with a broad range of concentrations of the tested calix[4]arenes. After 72 hours of treatment, the cytotoxicity was measured by means of the IC₅₀ value, using the kit "CellTiter 96R AQᵤₒᵤₛ One Solution Cell Proliferation Assay (MTS)" from Promega^{™}. Following 30 minutes of incubation with the MTS reagent, the endpoint measurements were done in triplicate at 485 nm, using a fluorescence plate reader.

The here below table 9 details the IC₅₀s using MTS cytotoxicity assay on H322 cells for the 6 below detailed calix[4]arenes of the invention:

**Table 9 of the IC₅₀s using MTS assay on H322 cells for 6 calix[4]arenes of the invention**

| **Compound of the formula/ Example** | **IC₅₀ (µM)** |
|---|---|
| (2a) / Example 1a | 5 |
| (5) / Example 2 | >10 |
| (3) / Example 3 | 5 |
| (6) / Example 4 | >10 |
| (7) / Example 5 | 5 |
| (4) / Example 7 | 6 |

Compounds with IC50> 10µM (Table 9) are considered inactive on H322 cancer cell line.

### VIII - EXPERIMENTS OF CYTOXICITY ON CALIX[4]ARENES OF THE INVENTION BASED ON DL₅₀:

Cytotoxicity was evaluated using the MTT assay. 1.10⁴ cells per well were seeded in a flat-bottom 96-well plate and were incubated for 48 hours at 37°C with 5% CO₂ before addition of the different calix[4]arenes at concentrations up to 200 µM. In all experiments, each condition was performed in triplicate in the plate. Besides, each experiment was done three times independently. After 24 hours of incubation in presence of the compounds, medium was discarded, cells were rinsed with PBS and 100 µL of medium containing 0.5 mg/mL of MTT was added to each well. After 30 minutes of incubation at 37°C, MTT solution was discarded and formazan crystals formed were dissolved in 150 µL of 4 mM HCl, 0.1 % NP40 in isopropanol. The absorbance in the wells were read in a microplate reader (Tecan microplate reader-550) at 570 nm.

The here below table 10 details the DL₅₀ on Hepatocellular carcinoma (HepG2/C3A) cells for:
- 10 calix[4]arenes of the invention,
- the comparative examples of the formulas (X) and (XI),
- disulfiram,
- the complex disulfiram/Cu²⁺ (1:1),
- clioquinol,
- the complex of clioquinol/Cu²⁺ (1:1).

As explained above, disulfiram and clioquinol are well-known ionophores for anticancer therapy.

**Table 10 detailing the DL₅₀ on Hepatocellular carcinoma (HepG2/C3A) cells for calix[4]arenes of the invention, the comparative examples and other ionophores**

| **Tested compound Formula** / **example** | **DL₅₀ (µM)** |
|---|---|
| (2) / example 1 | 3.1 |
| (2a) / example 1a | 4.0 |
| Disulfiram | 98.0 |
| Complex disulfiram/Cu (1:1) | 4.5 |
| Clioquinol | 67.0 |
| Complex clioquinol/Cu (1:1) | 24.0 |
| (6) / example 4 | 20.7 |
| (5) / example 2 | 23.0 |
| (3) / example 3 | 4.5 |
| (7) / example 5 | 10.9 |
| (8) / example 6 | 127.7 |
| (4) /example 7 | 3.1 |
| (9) / example 8 | 70.0 |
| (11) / example 10 | 140.0 |
| (X)/ 1^{st} comparative example | 148.7 |
| (XI) /2^{nd} comparative example | 184.0 |

In view of the table 10:
- The DL₅₀ values of the complexes clioquinol/Cu (1:1) and disulfiram/Cu (1:1) are lower than the DL₅₀ values of clioquinol and disulfiram. These two compounds are more toxic when they are in the form of a complex with Cu²⁺.
- In contrast, the calix[4]arenes of the present invention have the same toxicity alone or in complex with Cu⁺. DL₅₀ value of 3.1 versus 4.0 for the example 1.
- The calix[4]arenes of the formulas (2), (3) and (4) have the lowest DL₅₀ values. They are the most toxic calix[4]arenes.

### IX - EXPERIMENTS OF CYTOXICITY ON THE CALIX[4]ARENE OF THE FORMULA (2) IN COMBINATION WITH CuCl₂ :

### IX- A- Experiments on malignant H322 cells

H322 lung carcinoma cells were seeded in 96-well plates together with the calix[4]arene of the formula (2) and CuCl₂. Cells were grown in RPMI-1640 supplemented with GlutaMAX^{™} (Gibco^{™}) and fetal calf serum (FCS, 10%) treated with a broad range of concentrations of the calix[4]arene of the formula (2) or/and CuCl₂ from Merck. After 72 hours of treatment, the cytotoxicity was measured by means of the IC₅₀ value, using the kit "CellTiter 96R AQᵤₒᵤₛ One Solution Cell Proliferation Assay (MTS)" from Promega^{™}. Following 30 minutes of incubation with the MTS reagent, the endpoint measurements were done in triplicate at 485 nm, using a fluorescence plate reader.

The here below table 11 details the percentage of cell viability depending on the concentration of the calix[4]arene of the formula (2) and the concentration of Cu²⁺.

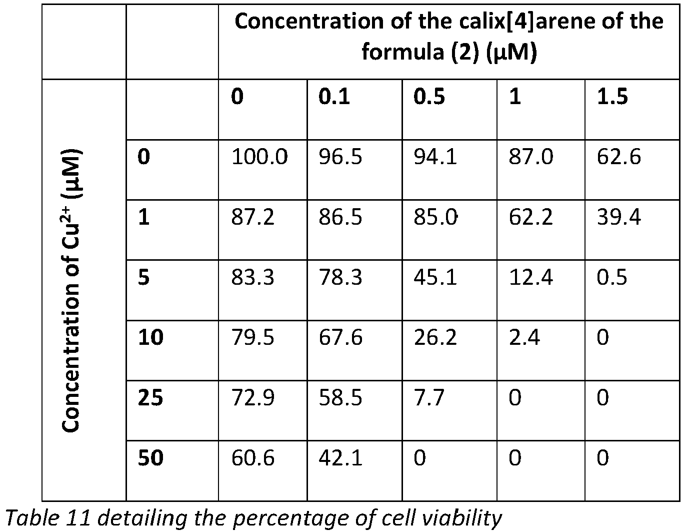

The table 11 details the effect of combining the calix[4]arene of the formula (2) with CuCl₂ using the MTS cytotoxicity assay in H322 cell line. More precisely, in view of the detailed percentages of cell viability in this table 11, it shows that the combination of the calix[4]arene of the formula (2) with Cu (in the form of CuCl₂) increases the efficacy of said calix(4]arene of the formula (2) in H322 cell line.

### IX- B - Experiments on malignant A549 cells

A549 human lung cancer cells were seeded in 96-well plates together with the calix[4]arene of the formula (2) and CuCl₂. Cells were grown in RPMI-1640 supplemented with GlutaMAX^{™} (Gibco^{™}) and fetal calf serum (FCS, 10%) treated with a broad range of concentrations of the calix[4]arene of the formula (2) or/and CuCb from Merck. After 72 hours of treatment, the cytotoxicity was measured by means of the IC₅₀ value, using the kit "CellTiter 96R AQᵤₒᵤₛ One Solution Cell Proliferation Assay (MTS)" from Promega^{™}. Following 30 minutes of incubation with the MTS reagent, the endpoint measurements were done in triplicate at 485 nm, using a fluorescence plate reader.

The here below table 12 details the percentage of cell viability depending on the concentration of the calix[4]arene of the formula (2) and the concentration of Cu²⁺.

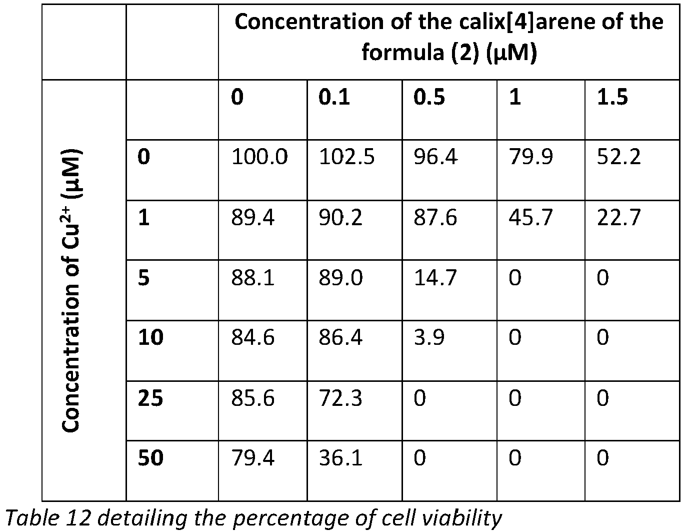

The table 12 details the effect of combining the calix[4]arene of the formula (2) with CuCl₂ using the MTS cytotoxicity assay in A549 cells. More precisely, in view of the detailed percentages of cell viability in this table 12, it shows that the combination of the calix[4]arene of the formula (2) with Cu (in the form of CuCl₂) increases the efficacy of said calix(4]arene of the formula (2) in A549 cells.

### IX- C - Experiments on malignant PC9 cells

PC9 human lung cancer cells were seeded in 96-well plates together with the calix[4]arene of the formula (2) and CuCl₂. Cells were grown in RPMI-1640 supplemented with GlutaMAX^{™} (Gibco^{™}) and fetal calf serum (FCS, 10%) treated with a broad range of concentrations of the calix[4]arene of the formula (2) or/and CuCl₂ from Merck. After 72 hours of treatment, the cytotoxicity was measured by means of the IC₅₀ value, using the kit "CellTiter 96R AQᵤₒᵤₛ One Solution Cell Proliferation Assay (MTS)" from Promega^{™}. Following 30 minutes of incubation with the MTS reagent, the endpoint measurements were done in triplicate at 485 nm, using a fluorescence plate reader.

The here below table 13 details the percentage of cell viability depending on the concentration of the calix[4]arene of the formula (2) and the concentration of Cu²⁺.

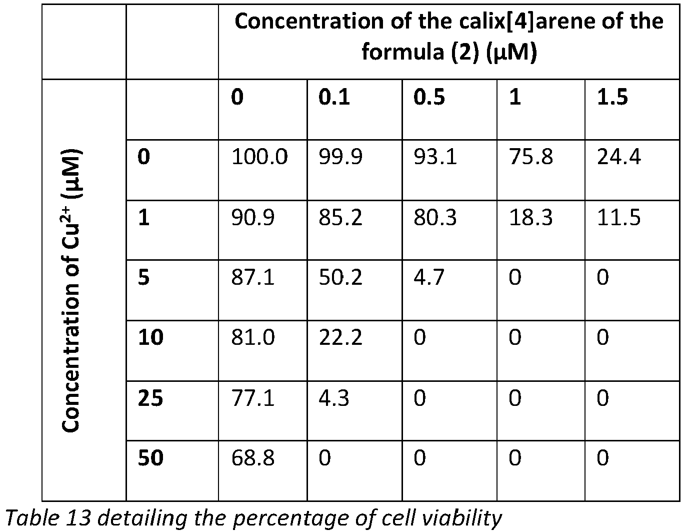

The table 13 details the effect of combining the calix[4]arene of the formula (2) with CuCl₂ using the MTS cytotoxicity assay in PC9 cells. More precisely, in view of the detailed percentages of cell viability in this table 13, it shows that the combination of the calix[4]arene of the formula (2) with Cu (in the form of CuCl₂) increases the efficacy of said calix(4]arene of the formula (2) in PC9 cells.

In conclusion, In view of these tables 11 to 13, the combination of the calix[4]arene of the formula (2) with Cu (in the form of CuCl₂) increases the efficacy of said calix(4]arene of the formula (2) in different lung adenocarcinoma cell lines. Thus we observe a synergistic effect of the calix[4]arene of the formula (2) with Cu in the form of a salt.

## Claims

1. A compound of the formula (1), 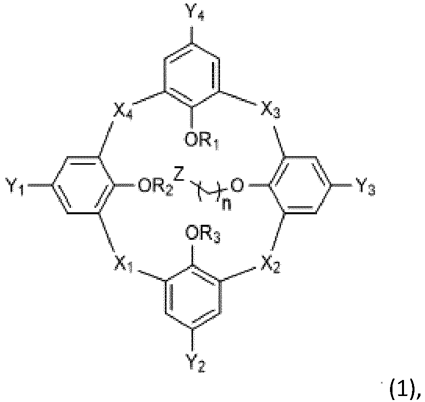
or a physiologically tolerable salt thereof
in which:
Y₁, Y₂, Y₃ and Y₄ are each independently selected from the group consisting of hydrogen, NO₂, amine, amide, azide, halogen, cyano, carboxylic acid ester, triazole and C₁-C₈-alkyl (preferably C₁-C₄-alkyl) group, said C₁-C₈-alkyl (preferably C₁-C₄-alkyl) group being optionally substituted with one or more substituents each independently selected from the group consisting of alkynyl, alkene, azide, ether, halogen, carboxylic acid ester, amide, cyano and triazole,
with the proviso that no one of Y₁, Y₂, Y₃ and Y₄ are a *tert*-butyl group, X₁, X₂, X₃ and X₄ are each independently selected from the group consisting of CH₂ and S, Z is a heterocyclic moiety which is selected from the group consisting of imidazole, benzimidazole, benzothiazole, benzoxazole, purine, oxazole, triazole, pyrazole and thiazole, said heterocyclic moiety being optionally substituted with 1 to 5 substituents which are selected from the group consisting of hydrogen, halogen, alkyl, alkenyl, alkynyl, phenyl, benzyl, OH, ether, ester, amide, NO₂, amine, azide and nitrile, R₁, R₂ and R₃ are each independently selected from the group consisting of -(CH₂)ₙZ, hydrogen and C₁-C₈-alkyl (preferably C₁₋C₃-alkyl) group, said C₁-C₈-alkyl group (preferably C₁-C₃-alkyl group) being optionally substituted with one or more substituents each independently selected from the group consisting of carboxylic acid ester, alkenyl, alkynyl, phenyl, OH, azide, amide, halogen, SH and thioether group,
n being an integer comprised between 1 and 4 (preferably equal to 1),
with the proviso that when R₁ and R₃ are each hydrogen and n is equal to 1, Z is not a benzothiazole group.

2. The compound or salt as claimed in claim 1, wherein Y₁, Y₂, Y₃ and Y₄ are each hydrogen or Y₂ and Y₄ are NO₂ and Y₁ and Y₃ are hydrogen.

3. The compound or salt as claimed in claim 1 or claim 2, wherein X₁, X₂, X₃ and X₄ are each CH₂.

4. The compound or salt as claimed in any one of claims 1 to 3, wherein R₂ is -(CH₂)ₙZ.

5. The compound or salt as claimed in any one of claims 1 to 4, wherein n is equal to 1.

6. The compound or salt as claimed in any one of claims 1 to 5, wherein Z is 1-methylimidazole, 1-ethylimidazole, 1-propylimidazole, 1-isopropylimidazole or a 1-benzylimidazole group.

7. The compound or salt as claimed in any one of claims 1 to 3, wherein R₁ and R₃ are each ester groups and R₂ is -(CH₂)ₙZ with n an integer comprised between 1 and 4 and Z is a substituted or unsubstituted imidazole group.

8. The compound or salt as claimed in claim 1, wherein X₁, X₂, X₃ and X₄ are each CH₂, Y₁, Y₂, Y₃ and Y₄ are each hydrogen, R₁ and R₃ are each selected from the group consisting of hydrogen, C₁-C₆-alkyl, propene and ethyl acetate group, R₂ is -(CH₂)ₙZ and Z is selected from the group consisting of 1-methylimidazole, 1-methylbenzimidazole, 9-methylpurine, oxazole and thiazole group with n being an integer comprised between 1 and 4 (preferably equal to 1).

9. The compound or salt as claimed in claim 8, wherein Z is 1-methylimidazole with n being an integer comprised between 1 and 4 (preferably equal to 1).

10. The compound or salt as claimed in claim 1 of the formula (2) or (3) or (4):

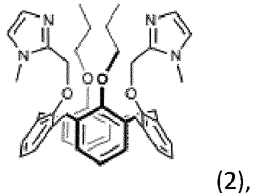

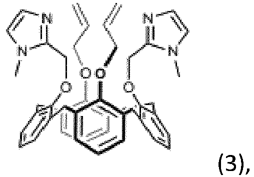

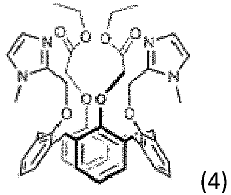

11. A 1:1 complex formed from a calix[4]arene or a physiologically tolerable salt thereof as claimed in any one of claims 1 to 10 and a cation, preferably Cu⁺.

12. A pharmaceutical composition, **characterized in that** it comprises at least one calix[4]arene of the formula (1) 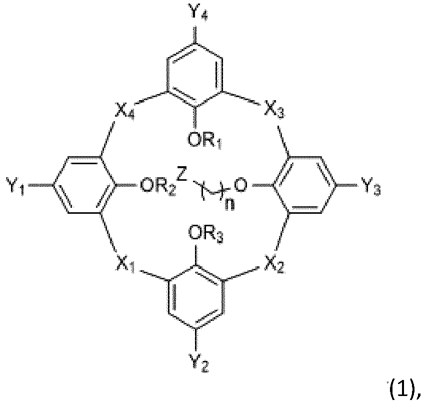 or a physiologically tolerable salt thereof or a 1:1 complex formed from a calix[4]arene of the formula (1) or from a physiologically tolerable salt thereof and a cation (preferably Cu⁺) in which:
Y₁, Y₂, Y₃ and Y₄ are each independently selected from the group consisting of hydrogen, NO₂, amine, amide, azide, halogen, cyano, carboxylic acid ester, triazole and C₁-C₈-alkyl (preferably C₁-C₄-alkyl) group, said C₁-C₈-alkyl (preferably C₁-C₄-alkyl) group being optionally substituted with one or more substituents each independently selected from the group consisting of alkynyl, alkene, azide, ether, halogen, carboxylic acid ester, amide, cyano and triazole,
with the proviso that no one of Y₁, Y₂, Y₃ and Y₄ are a *tert*-butyl group, X₁, X₂, X₃ and X₄ are each independently selected from the group consisting of CH₂ and S, Z is a heterocyclic moiety which is selected from the group consisting of imidazole, benzimidazole, benzothiazole, benzoxazole, pyridine, purine, oxazole, pyrazole, thiazole, triazole, pyrimidine, pyrazine and pyridazine, said heterocyclic moiety being optionally substituted with 1 to 5 substituents which are selected from the group consisting of hydrogen, halogen, alkyl, alkenyl, alkynyl, phenyl, benzyl, imidazole, benzimidazole, benzothiazole, benzoxazole, pyridine, purine, oxazole, pyrazole, thiazole, triazole, pyrimidine, pyrazine, pyridazine, OH, SH, thioether, ether, ester, amide, NO₂, amine, azide and nitrile,
R₁, R₂ and R₃ are each independently selected from the group consisting of -(CH₂)ₙZ, hydrogen and C₁-C₈-alkyl (preferably C₁₋C₃-alkyl) group, said C₁-C₈-alkyl group (preferably C₁-C₃-alkyl group) being optionally substituted with one or more substituents each independently selected from the group consisting of carboxylic acid ester, alkenyl, alkynyl, phenyl, OH, azide, cyano, amide, halogen, SH and thioether group,
n being an integer comprised between 1 and 8 (preferably equal to 1),
and together with at least one pharmaceutically acceptable carrier.

13. The pharmaceutical composition of claim 12, **characterized in that** the pharmaceutical composition further comprises at least one compound selected from the group consisting of chemotherapy compounds, immunotherapy compounds, antiviral compounds and antibiotics.

14. A calix[4]arene of the formula (1) or a physiologically tolerable salt thereof or a 1:1 complex formed from said calix[4]arene of the formula (1) or from said physiologically tolerable salt thereof and a cation (preferably Cu⁺) as defined in any one of claims 1 to 12 for use in the treatment of a disease selected from the group consisting of cancer or metastasis thereof, viral, bacterial, parasitic and fungal infections.
